(19) Europäisches Patentamt
European Patent Office
Office européen des brevets

(11) EP 2 325 234 B1

(12) # EUROPEAN PATENT SPECIFICATION

(45) Date of publication and mention of the grant of the patent:
**29.03.2017 Bulletin 2017/13**

(51) Int Cl.:
***C08G 75/02*** *(2016.01)* ***C08G 75/10*** *(2006.01)*
***C07D 339/06*** *(2006.01)* ***C07D 339/08*** *(2006.01)*

(21) Application number: **11001941.1**

(22) Date of filing: **03.12.2003**

(54) **HIGH IMPACT POLY(URETHANE UREA) POLYSULFIDES**

POLY(URETHAN-HARNSTOFF)-POLYSULFIDE MIT HOHER SCHLAGFESTIGKEIT

POLYSULFURES DE POLY(URÉTHANE URÉE) À HAUTE RESISTANCE AU CHOC

(84) Designated Contracting States:
**AT BE BG CH CY CZ DE DK EE ES FI FR GB GR HU IE IT LI LU MC NL PT RO SE SI SK TR**

(30) Priority: **20.12.2002 US 435537 P**
**02.12.2003 US 725023**

(43) Date of publication of application:
**25.05.2011 Bulletin 2011/21**

(62) Document number(s) of the earlier application(s) in accordance with Art. 76 EPC:
**03814645.2 / 1 581 574**

(73) Proprietor: **PPG Industries Ohio, Inc.**
**Cleveland, OH 44111 (US)**

(72) Inventors:
- **Bojkova, Nina V.**
**Monroeville**
**Pennsylvania 15146 (US)**
- **McDonald, William H.**
**Lower Burrell, PA 15068**
**Westmoreland County (US)**
- **Graham, Marvin J.**
**Monroeville, PA 15146 (US)**
- **Okoroafor, Michael O.**
**Roswell, Georgia 30075**
**Fulton County (US)**
- **Rao, Chandra Bhushan**
**Valencia, CA 91355 (US)**
- **Smith, Robert A.**
**Murrysville, PA 15668 (US)**
- **Herold, Robert D.**
**Monroeville, PA 15146 (US)**
- **Nagpal, Vidhu J.**
**Murrysville, PA 15668 (US)**
- **Yu, Phillip C.**
**Pittsburgh, PA 15239 (US)**
- **Sawant, Suresh**
**Stevenson Ranch, CA 91381 (US)**

(74) Representative: **f & e patent**
**Fleischer, Engels & Partner mbB, Patentanwälte**
**Braunsberger Feld 29**
**51429 Bergisch Gladbach (DE)**

(56) References cited:
**EP-A1- 1 384 736**
**EP-A1- 1 426 392**
**WO-A1-00/14137**
**WO-A1-01/36507**
**WO-A1-01/36508**
**WO-A1-03/042270**
**WO-A1-03/044070**
**US-A- 4 169 175**
**US-A- 5 574 124**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 325 234 B1

## Description

The present invention relates to sulfur-containing polyureaurethanes and methods for their preparation.

A number of organic polymeric materials, such as plastics, have been developed as alternatives and replacements for glass in applications such as optical lenses, fiber optics, windows and automotive, nautical and aviation transparencies. These polymeric materials can provide advantages relative to glass, including, shatter resistance, lighter weight for a given application, ease of molding and ease of dying. However, the refractive indices of many polymeric materials are generally lower than that of glass. In ophthalmic applications, the use of a polymeric material having a lower refractive index will require a thicker lens relative to a material having a higher refractive index. A thicker lens is not desirable.

Thus, there is a need in the art to develop a polymeric material having an adequate refractive index and good impact resistance/strength.

The present invention is directed to sulfur-containing polyureaurethanes as defined by claims 1 and 2.

At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective testing measurements.

As used herein and the claims, the term "cyanate" refers to isocyanate materials and isothiocyanate materials that are unblocked and capable of forming a covalent bond with a reactive group such as a thiol, hydroxyl, or amine function group. In a non-limiting embodiment, the polycyanate of the present invention can contain at least two functional groups chosen from isocyanate (NCO), isothiocyanate (NCS), and combinations of isocyanate and isothiocyanate functional groups. The term "isocyanate" refers to a cyanate which is free of sulfur. The term "isothiocyanate" refers to a sulfur-containing cyanate.

In alternate non-limiting embodiments, the polyureaurethane of the invention when polymerized can produce a polymerizate having a refractive index of at least 1.57, or at least 1.58, or at least 1.60, or at least 1.62. In further alternate non-limiting embodiments, the polyureaurethane of the invention when polymerized can produce a polymerizate having an Abbe number of at least 32, or at least 35, or at least 38, or at least 39, or at least 40, or at least 44. The refractive index and Abbe number can be determined by methods known in the art such as American Standard Test Method (ASTM) Number D 542-00. Further, the refractive index and Abbe number can be determined using various known instruments. In a non-limiting embodiment of the present invention, the refractive index and Abbe number can be measured in accordance with ASTM D 542-00 with the following exceptions: (i) test one to two samples/specimens instead of the minimum of three specimens specified in Section 7.3; and (ii) test the samples unconditioned instead of conditioning the samples/specimens prior to testing as specified in Section 8.1. Further, in a non-limiting embodiment, an Atago, model DR-M2 Multi-Wavelength Digital Abbe Refractometer can be used to measure the refractive index and Abbe number of the samples/specimens.

In alternate non-limiting embodiments, the amount of polycyanate and the amount of active hydrogen-containing material can be selected such that the equivalent ratio of (NCO + NCS):(SH + OH) can be greater than 1.0:1.0, or at least 2.0:1.0, or at least 2.5:1, or less than 4.5:1.0, or less than 5.5:1.0. In further alternate non-limiting embodiments, the equivalent ratio of (NCO + NCS)(SH + OH + NR), wherein R can be hydrogen or alkyl, can be greater than 1.0:1.0, or at least 2.0:1.0, or at least 2.5:1, or less than 4.5:1.0, or less than 5.5:1.0.

Polycyanates useful in the preparation of the polyureaurethane of the present invention are numerous and widely varied. Suitable polycyanates for use in the present invention can include but are not limited to polymeric and $C_2$-$C_{20}$ linear, branched, cyclic and aromatic polycyanates. Non-limiting examples can include polyisocyanates and polyisothiocyanates having backbone linkages chosen from urethane linkages (-NH-C(O)-O-), thiourethane linkages (-NH-C(O)-S-), thiocarbamate linkages (-NH-C(S)-O-), dithiourethane linkages (-NH-C(S)-S-) and combinations thereof.

The molecular weight of the polycyanate can vary widely. In alternate non-limiting embodiments, the number average molecular weight (Mn) can be at least 100 g/mole, or at least 150 g/mole, or less than 15,000 g/mole, or less than 5000 g/mole. The number average molecular weight can be determined using known methods. The number average molecular weight values recited herein and the claims were determined by gel permeation chromatography (GPC) using polystyrene standards.

Non-limiting examples of suitable polycyanates can include but are not limited to polyisocyanates having at least two isocyanate groups; isothiocyanates having at least two isothiocyanate groups; mixtures thereof; and combinations thereof, such as a material having isocyanate and isothiocyanate functionality.

Non-limiting examples of polyisocyanates can include but are not limited to aliphatic polyisocyanates, cycloaliphatic polyisocyanates wherein one or more of the isocyanato groups are attached directly to the cycloaliphatic

ring, cycloaliphatic polyisocyanates wherein one or more of the isocyanato groups are not attached directly to the cycloaliphatic ring, aromatic polyisocyanates wherein one or more of the isocyanato groups are attached directly to the aromatic ring, and aromatic polyisocyanates wherein one or more of the isocyanato groups are not attached directly to the aromatic ring. When an aromatic polyisocyanate is used, generally care should be taken to select a material that does not cause the polyureaurethane to color (e.g., yellow).

In a non-limiting embodiment of the present invention, the polyisocyanate can include but is not limited to aliphatic or cycloaliphatic diisocyanates, aromatic diisocyanates, cyclic dimmers and cyclic trimers thereof, and mixtures thereof. Non-limiting examples of suitable polyisocyanates can include but are not limited to Desmodur N 3300 (hexamethylene diisocyanate trimer) which is commercially available from Bayer; Desmodur N 3400 (60% hexamethylene diisocyanate dimer and 40% hexamethylene diisocyanate trimer).

In a non-limiting embodiment, the polyisocyanate can include dicyclohexylmethane diisocyanate and isomeric mixtures thereof. As used herein and the claims, the term "isomeric mixtures" refers to a mixture of the cis-cis, trans-trans, and cis-trans isomers of the polyisocyanate. Non-limiting examples of isomeric mixtures for use in the present invention can include the trans-trans isomer of 4,4'-methylenebis(cyclohexyl isocyanate), hereinafter referred to as "PICM" (paraisocyanato cyclohexylmethane), the cis-trans isomer of PICM, the cis-cis isomer of PICM, and mixtures thereof.

In one non-limiting embodiment, three suitable isomers of 4,4'-methylenebis(cyclohexyl isocyanate) for use in the present invention are shown below.

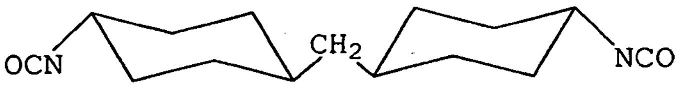

trans, trans

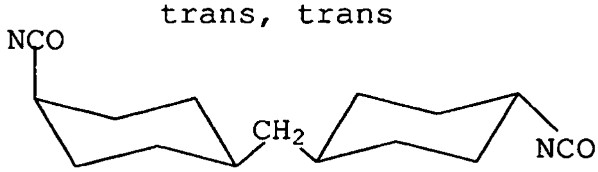

cis, trans

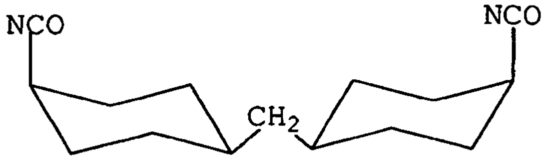

cis, cis

In one non-limiting embodiment, the PICM used in this invention can be prepared by phosgenating the 4,4'-methylenebis(cyclohexyl amine) (PACM) by procedures well known in the art such as the procedures disclosed in United States Patents 2,644,007 and 2,680,127. The PACM isomer mixtures, upon phosgenation, can produce PICM in a liquid phase, a partially liquid phase, or a solid phase at room temperature. The PACM isomer mixtures can be obtained by the hydrogenation of methylenedianiline and/or by fractional crystallization of PACM isomer mixtures in the presence of water and alcohols such as methanol and ethanol.

In a non-limiting embodiment, the isomeric mixture can contain from 10-100 percent of the trans,trans isomer of 4,4'-methylenebis(cyclohexyl isocyanate)(PICM).

Additional aliphatic and cycloaliphatic diisocyanates that can be used in alternate non-limiting embodiments of the present invention include 3-isocyanato-methyl-3,5,5-trimethyl cyclohexyl-isocyanate ("IPDI") which is commercially available from Arco Chemical, and meta-tetramethylxylene diisocyanate (1,3-bis(1-isocyanato-1-methylethyl)-benzene) which is commercially available from Cytec Industries Inc. under the tradename TMXDI.RTM. (Meta) Aliphatic Isocyanate.

As used herein and the claims, the terms aliphatic and cycloaliphatic diisocyanates refer to 6 to 100 carbon atoms linked in a straight chain or cyclized having two diisocyanate reactive end groups. In a non-limiting embodiment of the present invention, the aliphatic and cycloaliphatic diisocyanates for use in the present invention can include TMXDI and compounds of the formula $R-(NCO)_2$ wherein R represents an aliphatic group or a cycloaliphatic group.

Further non-limiting examples of suitable polycyanates can include but are not limited to aliphatic polyisocyanates and polyisothiocyanates; ethylenically unsaturated polyisocyanates and polyisothiocyanates; alicyclic polyisocyanates and polyisothiocyanates; aromatic polyisocyanates and polyisothiocyanates wherein the isocyanate groups

are not bonded directly to the aromatic ring, e.g., α,α'-xylene diisocyanate; aromatic polyisocyanates and polyisothiocyanates wherein the isocyanate groups are bonded directly to the aromatic ring, e.g., benzene diisocyanate; aliphatic polyisocyanates and polyisothiocyanates containing sulfide linkages; aromatic polyisocyanates and polyisothiocyanates containing sulfide or disulfide linkages; aromatic polyisocyanates and polyisothiocyanates containing sulfone linkages; sulfonic ester-type polyisocyanates and polyisothiocyanates, e.g., 4-methyl-3-isocyanatobenzenesulfonyl-4'-isocyanatophenol ester; aromatic sulfonic amide-type polyisocyanates and polyisothiocyanates; sulfur-containing heterocyclic polyisocyanates and polyisothiocyanates, e.g., thiophene-2,5-diisocyanate; halogenated, alkylated, alkoxylated, nitrated, carbodiimide modified, urea modified and biuret modified derivatives of polycyanates thereof; and dimerized and trimerized products of polycyanates thereof.

In a further non-limiting embodiment, a material of the following general formula (I) can be used in preparation of the polyurethane prepolymer:

(I) 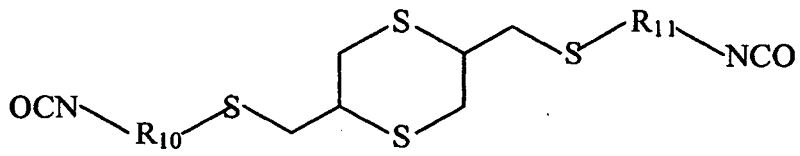

wherein $R_{10}$ and $R_{11}$ are each independently $C_1$ to $C_3$ alkyl.

Further non-limiting examples of aliphatic polyisocyanates can include ethylene diisocyanate, trimethylene diisocyanate, tetramethylene diisocyanate, hexamethylene diisocyanate, octamethylene diisocyanate, nonamethylene diisocyanate, 2,2'-dimethylpentane diisocyanate, 2,2,4-trimethylhexane diisocyanate, decamethylene diisocyanate, 2,4,4,-trimethylhexamethylene diisocyanate, 1,6,11-undecanetriisocyanate, 1,3,6-hexamethylene triisocyanate, 1,8-diisocyanato-4-(isocyanatomethyl)octane, 2,5,7-trimethyl-1,8-diisocyanato-5-(isocyanatomethyl)octane, bis(isocyanatoethyl)-carbonate, bis(isocyanatoethyl)ether, 2-isocyanatopropyl-2,6-diisocyanatohexanoate, lysinediisocyanate methyl ester and lysinetriisocyanate methyl ester.

Examples of ethylenically unsaturated polyisocyanates can include but are not limited to butene diisocyanate and 1,3-butadiene-1,4-diisocyanate. Alicyclic polyisocyanates can include but are not limited to isophorone diisocyanate, cyclohexane diisocyanate, methylcyclohexane diisocyanate, bis(isocyanatomethyl) cyclohexane, bis(isocyanatocyclohexyl)methane, bis(isocyanatocyclohexyl)-2,2-propane, bis(isocyanatocyclohexyl)-1,2-ethane, 2-isocyanatomethyl-3-(3-isocyanatopropyl)-5-isocyanatomethyl-bicyclo[2.2.1]-heptane, 2-isocyanatomethyl-3-(3-isocyanatopropyl)-6-isocyanatomethyl-bicyclo[2.2.1]-heptane, 2-isocyanatomethyl-2-(3-isocyanatopropyl)-5-isocyanatomethyl-bicyclo[2.2.1]-heptane, 2-isocyanatomethyl-2-(3-isocyanatopropyl)-6-isocyanatomethyl-bicyclo[2.2.1]-heptane, 2-isocyanatomethyl-3-(3-isocyanatopropyl)-6-(2-isocyanatoethyl)-bicyclo[2.2.1]-heptane, 2-isocyanatomethyl-2-(3-isocyanatopropyl)-5-(2-isocyanatoethyl)-bicyclo[2.2.1]-heptane and 2-isocyanatomethyl-2-(3-isocyanatopropyl)-6-(2-isocyanatoethyl)-bicyclo[2.2.1]-heptane.

Examples of aromatic polyisocyanates wherein the isocyanate groups are not bonded directly to the aromatic ring can include but are not limited to bis(isocyanatoethyl)benzene, α,α,α',α'-tetramethylxylene diisocyanate, 1,3-bis(1-isocyanato-1-methylethyl)benzene, bis(isocyanatobutyl)benzene, bis(isocyanatomethyl)naphthalene, bis(isocyanatomethyl)diphenyl ether, bis(isocyanatoethyl) phthalate, mesitylene triisocyanate and 2,5-di(isocyanatomethyl)furan. Aromatic polyisocyanates having isocyanate groups bonded directly to the aromatic ring can include but are not limited to phenylene diisocyanate, ethylphenylene diisocyanate, isopropylphenylene diisocyanate, dimethylphenylene diisocyanate, diethylphenylene diisocyanate, diisopropylphenylene diisocyanate, trimethylbenzene triisocyanate, benzene triisocyanate, naphthalene diisocyanate, methylnaphthalene diisocyanate, biphenyl diisocyanate, ortho- toluidine diisocyanate, ortho-tolylidine diisocyanate, ortho-tolylene diisocyanate, 4,4'-diphenylmethane diisocyanate, bis(3-methyl-4-isocyanatophenyl)methane, bis(isocyanatophenyl)ethylene, 3,3'-dimethoxy-biphenyl-4,4'-diisocyanate, triphenylmethane triisocyanate, polymeric 4,4'-diphenylmethane diisocyanate, naphthalene triisocyanate, diphenylmethane-2,4,4'-triisocyanate, 4-methyldiphenylmethane-3,5,2',4',6'-pentaisocyanate, diphenylether diisocyanate, bis(isocyanatophenylether)ethyleneglycol, bis(isocyanatophenylether)-1,3-propyleneglycol, benzophenone diisocyanate, carbazole diisocyanate, ethylcarbazole diisocyanate and dichlorocarbazole diisocyanate.

Further non-limiting examples of aliphatic and cycloaliphatic diisocyanates that can be used in the present invention include 3-isocyanato-methyl-3,5,5-trimethyl cyclohexyl-isocyanate ("IPDI") which is commercially available from Arco Chemical, and meta-tetramethylxylene diisocyanate (1,3-bis(1-isocyanato-1-methylethyl)-benzene) which is commercially available from Cytec Industries Inc. under the tradename TMXDI.RTM. (Meta) Aliphatic Isocyanate.

In a non-limiting embodiment of the present invention, the aliphatic and cycloaliphatic diisocyanates for use in the present invention can include TMXDI and compounds of the formula $R\text{-}(NCO)_2$ wherein R represents an aliphatic group or a cycloaliphatic group.

Non-limiting examples of polyisocyanates can include aliphatic polyisocyanates containing sulfide linkages

such as thiodiethyl diisocyanate, thiodipropyl diisocyanate, dithiodihexyl diisocyanate, dimethylsulfone diisocyanate, dithiodimethyl diisocyanate, dithiodiethyl diisocyanate, dithiodipropyl diisocyanate and dicyclohexylsulfide-4,4'-diisocyanate. Non-limiting examples of aromatic polyisocyanates containing sulfide or disulfide linkages include but are not limited to diphenylsulfide-2,4'-diisocyanate, diphenylsulfide-4,4'-diisocyanate, 3,3'-dimethoxy-4,4'-diisocyanatodibenzyl thioether, bis(4-isocyanatomethylbenzene)-sulfide, diphenyldisulfide-4,4'-diisocyanate, 2,2'-dimethyldiphenyldisulfide-5,5'-diisocyanate, 3,3'-dimethyldiphenyldisulfide-5,5'-diisocyanate, 3,3'-dimethyldiphenyldisulfide-6,6'-diisocyanate, 4,4'-dimethyldiphenyldisulfide-5,5'-diisocyanate, 3,3'-dimethoxydiphenyldisulfide-4,4'-diisocyanate and 4,4'-dimethoxydiphenyldisulfide-3,3'-diisocyanate.

Non-limiting examples polyisocyanates can include aromatic polyisocyanates containing sulfone linkages such as diphenylsulfone-4,4'-diisocyanate, diphenylsulfone-3,3'-diisocyanate, benzidinesulfone-4,4'-diisocyanate, diphenylmethanesulfone-4,4'-diisocyanate, 4-methyldiphenylmethanesulfone-2,4'-diisocyanate, 4,4'-dimethoxydiphenylsulfone-3,3'-diisocyanate, 3,3'-dimethoxy-4,4'-diisocyanatodibenzylsulfone, 4,4'-dimethyldiphenylsulfone-3,3'-diisocyanate, 4,4'-di-tert-butyl-diphenylsulfone-3,3'-diisocyanate and 4,4'-dichlorodiphenylsulfone-3,3'-diisocyanate.

Non-limiting examples of aromatic sulfonic amide-type polyisocyanates for use in the present invention can include 4-methyl-3-isocyanato-benzene-sulfonylanilide-3'-methyl-4'-isocyanate, dibenzenesulfonyl-ethylenediamine-4,4'-diisocyanate, 4,4'-methoxybenzenesulfonyl-ethylenediamine-3,3'-diisocyanate and 4-methyl-3-isocyanato-benzene-sulfonylanilide-4-ethyl-3'-isocyanate.

In alternate non-limiting embodiments, the polyisothiocyanate can include aliphatic polyisothiocyanates; alicyclic polyisothiocyanates, such as but not limited to cyclohexane diisothiocyanates; aromatic polyisothiocyanates wherein the isothiocyanate groups are not bonded directly to the aromatic ring, such as but not limited to $\alpha,\alpha'$-xylene diisothiocyanate; aromatic polyisothiocyanates wherein the isothiocyanate groups are bonded directly to the aromatic ring, such as but not limited to phenylene diisothiocyanate; heterocyclic polyisothiocyanates, such as but not limited to 2,4,6-triisothicyanato-1,3,5-triazine and thiophene-2,5-diisothiocyanate; carbonyl polyisothiocyanates; aliphatic polyisothiocyanates containing sulfide linkages, such as but not limited to thiobis(3-isothiocyanatopropane); aromatic polyisothiocyanates containing sulfur atoms in addition to those of the isothiocyanate groups; halogenated, alkylated, alkoxylated, nitrated, carbodiimide modified, urea modified and biuret modified derivatives of these polyisothiocyanates; and dimerized and trimerized products of these polyisothiocyanates.

Non-limiting examples of aliphatic polyisothiocyanates include 1,2-diisothiocyanatoethane, 1,3-diisothiocyanatopropane, 1,4-diisothiocyanatobutane and 1,6-diisothiocyanatohexane. Non-limiting examples of aromatic polyisothiocyanates having isothiocyanate groups bonded directly to the aromatic ring can include but are not limited to 1,2-diisothiocyanatobenzene, 1,3-diisothiocyanatobenzene, 1,4-diisothiocyanatobenzene, 2,4-diisothiocyanatotoluene, 2,5-diisothiocyanato-m-xylene, 4.4'-diisothiocyanato-1,1'-biphenyl, 1,1'-methylenebis(4-isothiocyanatobenzene), 1,1'-methylenebis(4-isothiocyanato-2-methylbenzene), 1,1'-methylenebis(4-isothiocyanato-3-methylbenzene), 1,1'-(1,2-ethane-diyl)bis(4-isothiocyanatobenzene), 4,4'-diisothiocyanatobenzophenenone, 4,4'-diisothiocyanato-3,3'-dimethylbenzophenone, benzanilide-3,4'-diisothiocyanate, diphenylether-4,4'-diisothiocyanate and diphenylamine-4.4'-diisothiocyanate.

Suitable carbonyl polyisothiocyanates can include but are not limited to hexane-dioyl diisothiocyanate, nonanedioyl diisothiocyanate, carbonic diisothiocyanate, 1,3-benzenedicarbonyl diisothiocyanate, 1,4-benzenedicarbonyl diisothiocyanate and (2,2'-bipyridine)-4,4'-dicarbonyl diisothiocyanate. Non-limiting examples of aromatic polyisothiocyanates containing sulfur atoms in addition to those of the isothiocyanate groups, can include but are not limited to 1-isothiocyanato-4-[(2-isothiocyanato)sulfonyl]benzene, thiobis(4-isothiocyanatobenzene), sulfonylbis(4-isothiocyanatobenzene), sulfinylbis(4-isothiocyanatobenzene), dithiobis(4-isothiocyanatobenzene), 4-isothiocyanato-1-[(4-isothiocyanatophenyl)-sulfonyl]-2-methoxybenzene, 4-methyl-3-isothicyanatobenzene-sulfonyl-4'-isothiocyanate phenyl ester and 4-methyl-3-isothiocyanatobenzene-sulfonylanilide-3'-methyl-4'-isothiocyanate.

Non-limiting examples of polycyanates having isocyanate and isothiocyanate groups can include aliphatic, alicyclic, aromatic, heterocyclic, or contain sulfur atoms in addition to those of the isothiocyanate groups. Non-limiting examples of such polycyanates include but are not limited to 1-isocyanato-3-isothiocyanatopropane, 1-isocyanato-5-isothiocyanatopentane, 1-isocyanato-6-isothiocyanatohexane, isocyanatocarbonyl isothiocyanate, 1-isocyanato-4-isothiocyanatocyclohexane, 1-isocyanato-4-isothiocyanatobenzene, 4-methyl-3-isocyanato-1-isothiocyanatobenzene, 2-isocyanato-4,6-diisothiocyanato-1,3,5-triazine, 4-isocyanato-4'-isothiocyanato-diphenyl sulfide and 2-isocyanato-2'-isothiocyanatodiethyl disulfide.

Active hydrogen-containing materials are varied and known in the art. Non-limiting examples can include hydroxyl-containing materials such as but not limited to polyols; sulfur-containing materials such as but not limited to hydroxyl functional polysulfides, and SH-containing materials such as but not limited to polythiols; and materials having both hydroxyl and thiol functional groups.

Suitable hydroxyl-containing materials for use in the present invention can include a wide variety of materials known in the art. Non-limiting examples can include but are not limited to polyether polyols, polyester polyols, polycaprolactone polyols, polycarbonate polyols, and mixtures thereof.

Polyether polyols and methods for their preparation are known to one skilled in the art. Many polyether polyols of various types and molecular weight are commercially available from various manufacturers. Non-limiting examples of polyether polyols can include but are not limited to polyoxyalkylene polyols, and polyalkoxylated polyols. Polyoxyalkylene polyols can be prepared in accordance with known methods. In a non-limiting embodiment, a polyoxyalkylene polyol can be prepared by condensing an alkylene oxide, or a mixture of alkylene oxides, using acid- or base-catalyzed addition with a polyhydric initiator or a mixture of polyhydric initiators, such as but not limited to ethylene glycol, propylene glycol, glycerol, and sorbitol. Non-limiting examples of alkylene oxides can include ethylene oxide, propylene oxide, butylene oxide, amylene oxide, aralkylene oxides, such as but not limited to styrene oxide, mixtures of ethylene oxide and propylene oxide. In a further non-limiting embodiment, polyoxyalkylene polyols can be prepared with mixtures of alkylene oxide using random or stepwise oxyalkylation. Non-limiting examples of such polyoxyalkylene polyols include polyoxyethylene, such as but not limited to polyethylene glycol, polyoxypropylene, such as but not limited to polypropylene glycol.

In a non-limiting embodiment, polyalkoxylated polyols can be represent by the following general formula:

(I')

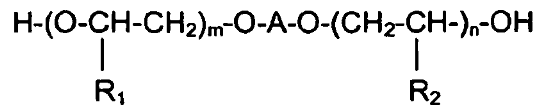

wherein m and n can each be a positive integer, the sum of m and n being from 5 to 70; $R_1$ and $R_2$ are each hydrogen, methyl or ethyl; and A is a divalent linking group such as a straight or branched chain alkylene which can contain from 1 to 8 carbon atoms, phenylene, and $C_1$ to $C_9$ alkyl-substituted phenylene. The chosen values of m and n can, in combination with the chosen divalent linking group, determine the molecular weight of the polyol. Polyalkoxylated polyols can be prepared by methods that are known in the art. In a non-limiting embodiment, a polyol such as 4,4'-isopropylidenediphenol can be reacted with an oxirane-containing material such as but not limited to ethylene oxide, propylene oxide and butylene oxide, to form what is commonly referred to as an ethoxylated, propoxylated or butoxylated polyol having hydroxy functionality. Non-limiting examples of polyols suitable for use in preparing polyalkoxylate polyols can include those polyols described in United States Patent 6,187,444 B1 at column 10, lines 1-20.

As used herein and the claims, the term "polyether polyols" can include the generally known poly(oxytetramethylene) diols prepared by the polymerization of tetrahydrofuran in the presence of Lewis acid catalysts such as but not limited to boron trifluoride, tin (IV) chloride and sulfonyl chloride. Also included are the polyethers prepared by the copolymerization of cyclic ethers such as but not limited to ethylene oxide, propylene oxide, trimethylene oxide, and tetrahydrofuran with aliphatic diols such as but not limited to ethylene glycol, 1,3-butanediol, 1,4-butanediol, diethylene glycol, dipropylene glycol, 1,2-propylene glycol and 1,3-propylene glycol. Compatible mixtures of polyether polyols can also be used. As used herein, "compatible" means that the polyols are mutually soluble in each other so as to form a single phase.

A variety of polyester polyols for use in the present invention are known in the art. Suitable polyester polyols can include but are not limited to polyester glycols. Polyester glycols for use in the present invention can include the esterification products of one or more dicarboxylic acids having from four to ten carbon atoms, such as but not limited to adipic, succinic or sebacic acids, with one or more low molecular weight glycols having from two to ten carbon atoms, such as but not limited to ethylene glycol, propylene glycol, diethylene glycol, 1,4-butanediol, neopentyl glycol, 1,6-hexanediol and 1,10-decanediol. Esterification procedures for producing polyester polyols is described, for example, in the article D.M. Young, F. Hostettler et al., "Polyesters from Lactone," Union Carbide F-40, p. 147.

In a non-limiting embodiment, the polyol for use in the present invention can include polycaprolactone polyols. Suitable polycaprolactone polyols are varied and known in the art. In a non-limiting embodiment, polycaprolactone polyols can be prepared by condensing caprolactone in the presence of difunctional active hydrogen compounds such as but not limited to water or low molecular weight glycols as recited herein. Non-limiting examples of suitable polycaprolactone polyols can include commercially available materials designated as the CAPA series from Solvay Chemical which includes but is not limited to CAPA 2047A, and the TONE series from Dow Chemical such as but not limited to TONE 0201.

Polycarbonate polyols for use in the present invention are varied and known to one skilled in the art. Suitable polycarbonate polyols can include those commercially available (such as but not limited to Ravecarb™ 107 from Enichem S.p.A.). In a non-limiting embodiment, the polycarbonate polyol can be produced by reacting an organic glycol such as a diol, described hereinafter and in connection with the glycol component of the polyureaurethane, and a dialkyl carbonate, such as described in United States Patent 4,160,853. In a non-limiting embodiment, the polyol can include polyhexamethyl carbonate such as $HO-(CH_2)_6-[O-C(O)-O-(CH_2)_6]_n-OH$, wherein n is an integer from 4 to 24, or from 4 to 10, or from 5 to 7.

In a non-limiting embodiment, the glycol material can comprise low molecular weight polyols such as polyols having a number average molecular weight of less than 500 g/mole, and compatible mixtures thereof. As used herein,

"compatible" means that the glycols are mutually soluble in each other so as to form a single phase. Non-limiting examples of these polyols can include but are not limited to low molecular weight diols and triols. In a further non-limiting embodiment, the amount of triol chosen is such to avoid a high degree of crosslinking in the polyurethane. The organic glycol typically contains from 2 to 16, or from 2 to 6, or from 2 to 10, carbon atoms. Non-limiting examples of such glycols can include but are not limited to ethylene glycol, propylene glycol, diethylene glycol, triethylene glycol, tetraethylene glycol, dipropylene glycol, tripropylene glycol, 1,2-, 1,3- and 1,4-butanediol, 2,2,4-trimethyl-1,3-pentanediol, 2-methyl-1,3-pentanediol, 1,3- 2,4- and 1,5-pentanediol, 2,5- and 1,6-hexanediol, 2,4-heptanediol, 2-ethyl-1,3-hexanediol, 2,2-dimethyl-1,3-propanediol, 1,8-octanediol, 1,9-nonanediol, 1,10-decanediol, 1,4-cyclohexanediol, 1,4-cyclohexanedimethanol, 1,2-bis(hydroxyethyl)-cyclohexane, glycerin, tetramethylolmethane, such as but not limited to pentaerythritol, trimethylolethane and trimethylolpropane; and isomers thereof.

In alternate non-limiting embodiments, the hydroxyl-containing material can have a molecular weight of at least 200 g/mole, or at least 1000 g/mole, or at least 2000 g/mole. In alternate non-limiting embodiments, the hydroxyl-containing material can have a number average molecular weight of less than 10,000 g/mole, or less than 15,000 g/mole, or less than 20,000 g/mole, or less than 32,000 g/mole.

In a non-limiting embodiment, the polyether-containing polyol material for use in the present invention can include teresters produced from at least one low molecular weight dicarboxylic acid, such as adipic acid.

Polyether glycols for use in the present invention can include but are not limited to polytetramethylene ether glycol.

In a non-limiting embodiment, the active hydrogen-containing material can comprise block polymers including blocks of ethylene oxide-propylene oxide and/or ethylene oxide-butylene oxide. In a non-limiting embodiment, the active hydrogen-containing material can comprise a block polymer of the following chemical formula:

(I'') $$H\text{-}(O\text{-}CRRCRR\text{-}Y_n)_a\text{-}(CRRCRR\text{-}Y_n\text{-}O)_b\text{-}(CRRCRR\text{-}Y_n\text{-}O)_c\text{-}H$$

wherein R can represent hydrogen or $C_1$-$C_6$ alkyl; $Y_n$ can represent $C_0$-$C_6$ hydrocarbon; n can be an integer from 0 to 6; a, b, and c can each be an integer from 0 to 300, wherein a, b and c are chosen such that the number average molecular weight of the polyol does not exceed 32,000 g/mole.

In a further non-limiting embodiment, Pluronic R, Pluronic L62D, Tetronic R and Tetronic, which are commercially available from BASF, can be used as the active hydrogen-containing material in the present invention.

Non-limiting examples of suitable polyols for use in the present invention include straight or branched chain alkane polyols, such as but not limited to 1,2-ethanediol, 1,3-propanediol, 1,2-propanediol, 1,4-butanediol, 1,3-butanediol, glycerol, neopentyl glycol, trimethylolethane, trimethylolpropane, di-trimethylolpropane, erythritol, pentaerythritol and dipentaerythritol; polyalkylene glycols, such as but not limited to diethylene glycol, dipropylene glycol and higher polyalkylene glycols such as but not limited to polyethylene glycols which can have number average molecular weights of from 200 g/mole to 2,000 g/mole; cyclic alkane polyols, such as but not limited to cyclopentanediol, cyclohexanediol, cyclohexanetriol, cyclohexanedimethanol, hydroxypropylcyclohexanol and cyclohexanediethanol; aromatic polyols, such as but not limited to dihydroxybenzene, benzenetriol, hydroxybenzyl alcohol and dihydroxytoluene; bisphenols, such as, 4,4'-isopropylidenediphenol; 4,4'-oxybisphenol, 4,4'-dihydroxybenzophenone, 4,4'-thiobisphenol, phenolphthlalein, bis(4-hydroxyphenyl)methane, 4,4'-(1,2-ethenediyl)bisphenol and 4,4'-sulfonylbisphenol; halogenated bisphenols, such as but not limited to 4,4'-isopropylidenebis(2,6-dibromophenol), 4,4'-isopropylidenebis(2,6-dichlorophenol) and 4,4'-isopropylidenebis(2,3,5,6-tetrachlorophenol); alkoxylated bisphenols, such as but not limited to alkoxylated 4,4'-isopropylidenediphenol which can have from 1 to 70 alkoxy groups, for example, ethoxy, propoxy, $\alpha$-butoxy and $\beta$-butoxy groups; and biscyclohexanols, which can be prepared by hydrogenating the corresponding bisphenols, such as but not limited to 4,4'-isopropylidene-biscyclohexanol, 4,4'-oxybiscyclohexanol, 4,4'-thiobiscyclohexanol and bis(4-hydroxycyclohexanol)methane; polyurethane polyols, polyester polyols, polyether polyols, poly vinyl alcohols, polymers containing hydroxy functional acrylates, polymers containing hydroxy functional methacrylates, and polymers containing allyl alcohols.

In a non-limiting embodiment, the polyol can be chosen from multifunctional polyols, including but not limited to trimethylolpropane, ethoxylated trimethylolpropane, pentaerythritol.

In a further non-limiting embodiment, the polyol can be a polyurethane prepolymer having two or more hydroxy functional groups. Such polyurethane prepolymers can be prepared from any of the above-listed polyols and aforementioned polyisocyanates. In a non-limiting embodiment, the OH:NCO equivalent ratio can be chosen such that essentially no free NCO groups are produced in preparing the polyurethane prepolymer. In a non-limiting embodiment, the equivalent ratio of NCO (i.e., isocyanate) to OH present in the polyether-containing polyurethane prepolymer can be an amount of from 2.0 to less than 5.5 NCO/1.0 OH.

In alternate non-limiting embodiments, the polyurethane prepolymer can have a number average molecular weight (Mn) of less than 50,000 g/mole, or less than 20,000 g/mole, or less than 10,000 g/mole. The Mn can be determined using a variety of known methods. In a non-limiting embodiment, the Mn can be determined by gel permeation chromatography (GPC) using polystyrene standards.

In a non-limiting embodiment, the sulfur-containing active hydrogen material for use in the present invention can include a SH-containing material such as but not limited to a polythiol having at least two thiol groups. Non-limiting examples of suitable polythiols can include but are not limited to aliphatic polythiols, cycloaliphatic polythiols, aromatic polythiols, heterocyclic polythiols, polymeric polythiols and mixtures thereof. The hydrogen-containing material can have linkages including but not limited to ether linkages (-O-), sulfide linkages (-S-), polysulfide linkages ($-S_x-$, wherein x is at least 2, or from 2 to 4) and combinations of such linkages. As used herein and the claims, the terms "thiol," "thiol group," "mercapto" or "mercapto group" refer to an -SH group which is capable of forming a thiourethane linkage, (i.e., -NH-C(O)-S-) with an isocyanate group or a dithioruethane linkage (i.e., -NH-C(S)-S-) with an isothiocyanate group.

Non-limiting examples of suitable polythiols can include but are not limited to 2,5-dimercaptomethyl-1,4-dithiane, dimercaptoethylsulfide, pentaerythritol tetrakis(3-mercaptopropionate), pentaerythritol tetrakis(2-mercaptoacetate), trimethylolpropane tris(3-mercaptopropionate), trimethylolpropane tris(2-mercaptoacetate), 4-mercaptomethyl-3,6-dithia-1,8-octanedithiol, 4-tert-butyl-1,2-benzenedithiol, 4,4'-thiodibenzenethiol, ethanedithiol, benzenedithiol, ethylene glycol di(2-mercaptoacetate), ethylene glycol di(3-mercaptopropionate), poly(ethylene glycol) di(2-mercaptoacetate) and poly(ethylene glycol) di(3-mercaptopropionate), and mixtures thereof.

In a non-limiting embodiment, the polythiol can be chosen from materials represented by the following general formula,

**(II)**

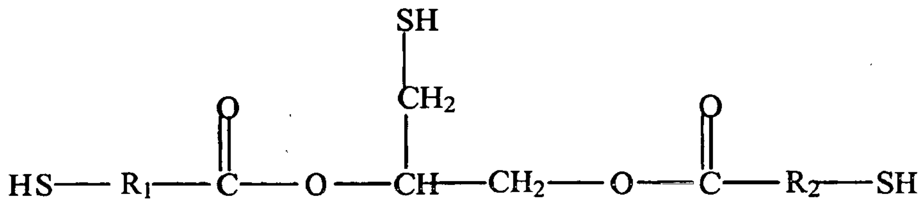

wherein $R_1$ and $R_2$ can each be independently chosen from straight or branched chain alkylene, cyclic alkylene, phenylene and $C_1$-$C_9$ alkyl substituted phenylene. Non-limiting examples of straight or branched chain alkylene can include but are not limited to methylene, ethylene, 1,3-propylene, 1,2-propylene, 1,4-butylene, 1,2-butylene, pentylene, hexylene, heptylene, octylene, nonylene, decylene, undecylene, octadecylene and icosylene. Non-limiting examples of cyclic alkylenes can include but are not limited to cyclopentylene, cyclohexylene, cycloheptylene, cyclooctylene, and alkyl-substituted derivatives thereof. In a non-limiting embodiment, the divalent linking groups $R_1$ and $R_2$ can be chosen from phenylene and alkyl-substituted phenylene, such as methyl, ethyl, propyl, isopropyl and nonyl substituted phenylene. In a further non-limiting embodiment, $R_1$ and $R_2$ are each methylene or ethylene.

The polythiol represented by general formula II can be prepared by any known method. In a non-limiting embodiment, the polythiol of formula (II) can be prepared from an esterification or transesterification reaction between 3-mercapto-1,2-propanediol (Chemical Abstract Service (CAS) Registry No. 96-27-5) and a thiol functional carboxylic acid or carboxylic acid ester in the presence of a strong acid catalyst, such as but not limited to methane sulfonic acid, with the concurrent removal of water or alcohol from the reaction mixture. A non-limiting example of a polythiol of formula II includes a structure wherein $R_1$ and $R_2$ are each methylene.

In a non-limiting embodiment, the polythiol represented by general formula II can be thioglycerol bis(2-mercaptoacetate). As used herein and the claims, the term "thioglycerol bis(2-mercaptoacetate)" refers to any related co-product oligomeric species and polythiol monomer compositions containing residual starting materials. In a non-limiting embodiment, oxidative coupling of thiol groups can occur when washing the reaction mixture resulting from the esterification of 3-mercapto-1,2-propanediol and a thiol functional carboxylic acid, such as but not limited to 2-mercaptoacetic acid, with excess base, such as but not limited to aqueous ammonia. Such an oxidative coupling can result in the formation of oligomeric polythiol species having disulfide linkages, such as but not limited to -S-S- linkages.

Suitable polythiols for use in the present invention can include but are not limited to polythiol oligomers having disulfide linkages, which can be prepared from the reaction of a polythiol having at least two thiol groups and sulfur in the presence of a basic catalyst. In a non-limiting embodiment, the equivalent ratio of polythiol monomer to sulfur can be from m to (m-1) wherein m can represent an integer from 2 to 21. The polythiol can be chosen from the above-mentioned examples, such as but not limited to 2,5-dimercaptomethyl-1,4-dithiane. In alternate non-limiting embodiments, the sulfur can be in the form of crystalline, colloidal, powder and sublimed sulfur, and can have a purity of at least 95 percent or at least 98 percent.

Non-limiting examples of co-product oligomeric species can include materials represented by the following general formula:

(III)

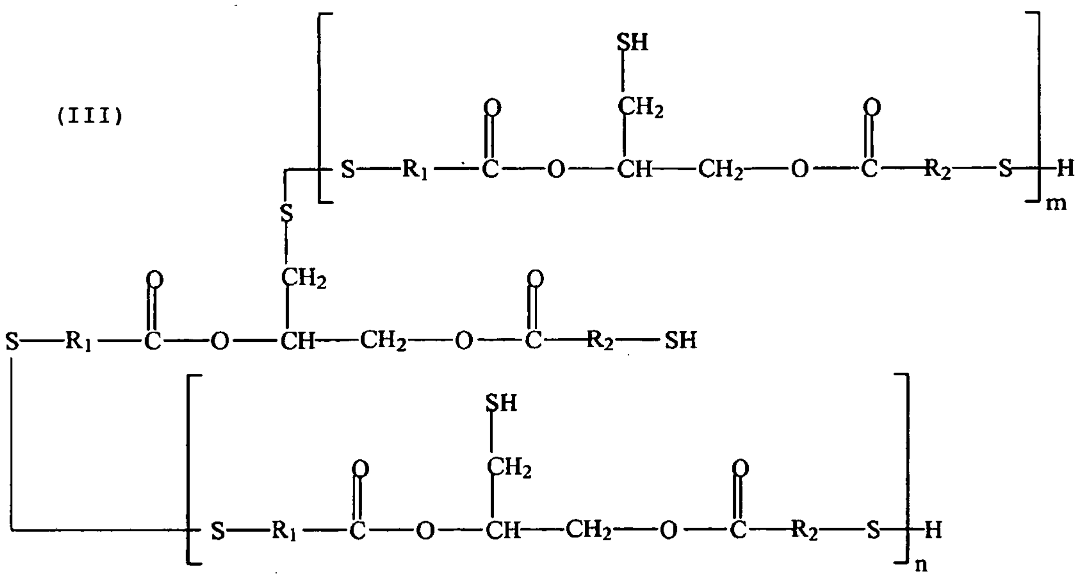

wherein $R_1$ and $R_2$ can be as described above, n and m can be independently an integer from 0 to 21 and (n + m) can be at least 1.

In another non-limiting embodiment, the polythiol oligomer can have disulfide linkages and can include materials represented by the following general formula IV,

(IV)

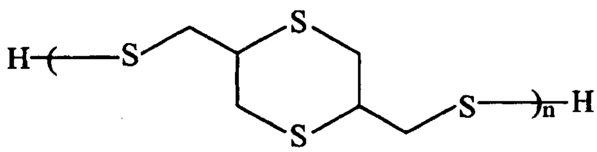

wherein n can represent an integer from 1 to 21. In a non-limiting embodiment, the polythiol oligomer represented by general formula IV can be prepared by the reaction of 2,5-dimeracaptomethyl-1,4-dithiane with sulfur in the presence of a basic catalyst, as described previously herein.

In a non-limiting embodiment, the polythiol for use in the present invention, can include at least one polythiol represented by the following structural formulas.

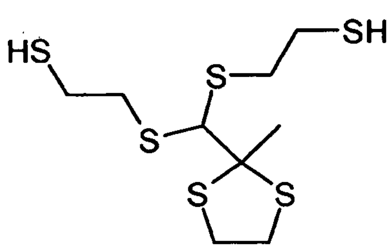

(IV'a)

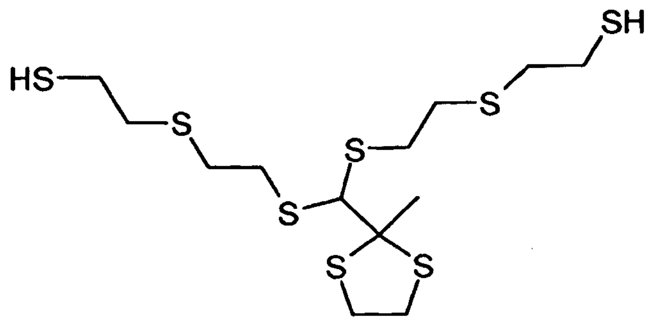

(IV'b)

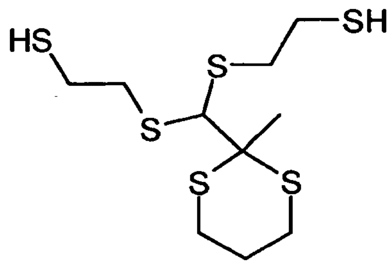

(IV'c)

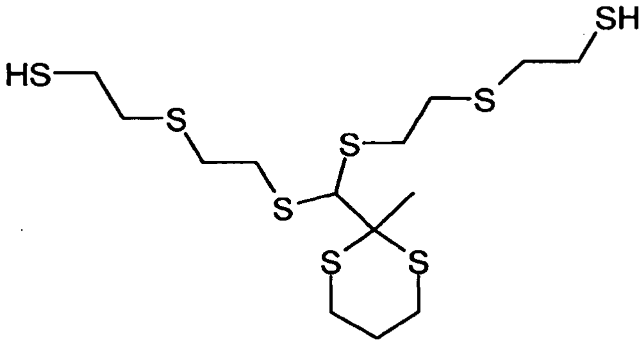

(IV'd)

The sulfide-containing polythiols comprising 1,3-dithiolane (e.g., formulas IV'a and b) or 1,3-dithiane (e.g., formulas IV'c and d) can be prepared by reacting asym-dichloroacetone with polymercaptan, and then reacting the reaction product with polymercaptoalkylsulfide, polymercaptan or mixtures thereof.

Non-limiting examples of suitable polymercaptans for use in the reaction with asym-dichloroacetone can include but are not limited to materials represented by the following formula,

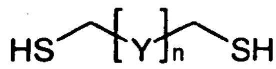

**1**

wherein Y can represent $CH_2$ or ($CH_2$-S-$CH_2$), and n can be an integer from 0 to 5. In a non-limiting embodiment, the polymercaptan for reaction with asym-dichloroacetone in the present invention can be chosen from ethanedithiol, propanedithiol, and mixtures thereof.

The amount of asym-dichloroacetone and polymercaptan suitable for carrying out the above reaction can vary. In a non-limiting embodiment, asym-dichloroacetone and polymercaptan can be present in the reaction mixture in an amount such that the molar ratio of dichloroacetone to polymercaptan can be from 1:1 to 1:10.

Suitable temperatures for reacting asym-dichloroacetone with polymercaptane can vary. In a non-limiting embodiment, the reaction of asym-dichloroacetone with polymercaptane can be carried out at a temperature within the range of from 0 to 100°C.

Non-limiting examples of suitable polymercaptans for use in the reaction with the reaction product of the asym-dichloroacetone and polymercaptan, can include but are not limited to materials represented by the above general formula 1, aromatic polymercaptans, cycloalkyl polymercaptans, heterocyclic polymercaptans, branched polymercaptans, and mixtures thereof.

Non-limiting examples of suitable polymercaptoalkylsulfides for use in the reaction with the reaction product of the asym-dichloroacetone and polymercaptan, can include but are not limited to materials represented by the following formula,

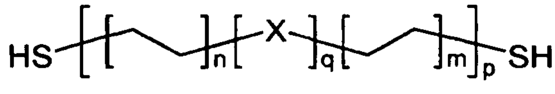

**2**

wherein X can represent O, S or Se, n can be an integer from 0 to 10, m can be an integer from 0 to 10, p can be an integer from 1 to 10, q can be an integer from 0 to 3, and with the proviso that (m + n) is an integer from 1 to 20.

Non-limiting examples of suitable polymercaptoalkylsulfides for use in the present invention can include branched polymercaptoalkylsulfides. In a non-limiting embodiment, the polymercaptoalkylsulfide for use in the present invention can be dimercaptoethylsulfide.

The amount of polymercaptan, polymercaptoalkylsulfide, or mixtures thereof, suitable for reacting with the reaction product of asym-dichloroacetone and polymercaptan, can vary. In a non-limiting embodiment, polymercaptan,

polymercaptoalkylsulfide, or a mixture thereof, can be present in the reaction mixture in an amount such that the equivalent ratio of reaction product to polymercaptan, polymercaptoalkylsulfide, or a mixture thereof, can be from 1:1.01 to 1:2. Moreover, suitable temperatures for carrying out this reaction can vary. In a non-limiting embodiment, the reaction of polymercaptan, polymercaptoalkylsulfide, or a mixture thereof, with the reaction product can be carried out at a temperature within the range of from 0 to 100°C.

In a non-limiting embodiment, the reaction of asym-dichloroacetone with polymercaptan can be carried out in the presence of an acid catalyst. The acid catalyst can be selected from a wide variety known in the art, such as but not limited to Lewis acids and Bronsted acids. Non-limiting examples of suitable acid catalysts can include those described in Ullmann's Encyclopedia of Industrial Chemistry, 5th Edition, 1992, Volume A21, pp. 673 to 674. In further alternate non-limiting embodiments, the acid catalyst can be chosen from boron trifluoride etherate, hydrogen chloride, toluenesulfonic acid, and mixtures thereof.

The amount of acid catalyst can vary. In a non-limiting embodiment, a suitable amount of acid catalyst can be from 0.01 to 10 percent by weight of the reaction mixture.

In another non-limiting embodiment, the reaction product of asym-dichloroacetone and polymercaptan can be reacted with polymercaptoalkylsulfide, polymercaptan or mixtures thereof, in the presence of a base. The base can be selected from a wide variety known in the art, such as but not limited to Lewis bases and Bronsted bases. Non-limiting examples of suitable bases can include those described in Ullmann's Encyclopedia of Industrial Chemistry, 5th Edition, 1992, Volume A21, pp. 673 to 674. In a further non-limiting embodiment, the base can be sodium hydroxide.

The amount of base can vary. In a non-limiting embodiment, a suitable equivalent ratio of base to reaction product of the first reaction, can be from 1:1 to 10:1.

In another non-limiting embodiment, the preparation of these sulfide-containing polythiols can include the use of a solvent. The solvent can be selected from a wide variety known in the art.

In a further non-limiting embodiment, the reaction of asym-dichloroacetone with polymercaptan can be carried out in the presence of a solvent. The solvent can be selected from a wide variety of known materials. In a non-limiting embodiment, the solvent can be selected from but is not limited to organic solvents, including organic inert solvents. Non-limiting examples of suitable solvents can include but are not limited to chloroform, dichloromethane, 1,2-dichloroethane, diethyl ether, benzene, toluene, acetic acid and mixtures therof. In still a further embodiment, the reaction of asym-dichloroacetone with polymercaptan can be carried out in the presence of toluene as solvent.

In another embodiment, the reaction product of asym-dichloroacetone and polymercaptan can be reacted with polymercaptoalkylsulfide, polymercaptan or mixtures thereof, in the presence of a solvent, wherein the solvent can be selected from but is not limited to organic solvents including organic inert solvents. Non-limiting examples of suitable organic and inert solvents can include alcohols such as but not limited to methanol, ethanol and propanol; aromatic hydrocarbon solvents such as but not limited to benzene, toluene, xylene; ketones such as but not limited to methyl ethyl ketone; water and mixtures thereof. In a further non-limiting embodiment, this reaction can be carried out in the presence of a mixture of toluene and water as solvent system. In another non-limiting embodiment, this reaction can be carried out in the presence of ethanol as solvent.

The amount of solvent can widely vary. In a non-limiting embodiment, a suitable amount of solvent can be from 0 to 99 percent by weight of the reaction mixture. In a further non-limiting embodiment, the reaction can be carried out neat, i.e., without solvent.

In another non-limiting embodiment, the reaction of asym-dichloroacetone with polyercaptan can be carried out in the presence of a dehydrating reagent. The dehydrating reagent can be selected from a wide variety known in the art. Suitable dehydrating reagents for use in this reaction can include but are not limited to magnesium sulfate. The amount of dehydrating reagent can vary widely according to the stoichiometry of the dehydrating reaction.

In a non-limiting embodiment, a sulfide-containing polythiol of the present invention can be prepared by reacting 1,1-dichloroacetone with 1,2-ethanedithiol to produce 2-methyl-2-dichloromethyl-1,3-dithiolane, as shown below.

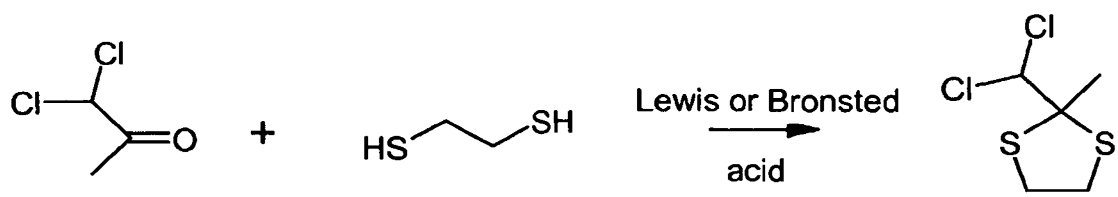


In a further non-limiting embodiment, 1,1-dichloroacetone can be reacted with 1,3-propanedithiol to produce a 2-methyl-2-dichloromethyl-1,3-dithiane, as shown below.

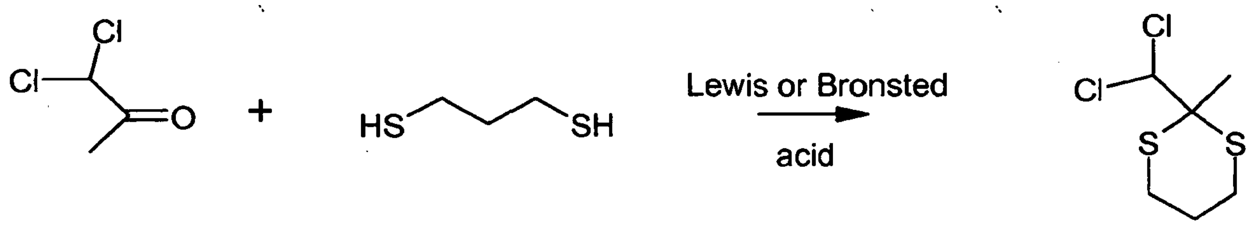


In another non-limiting embodiment, 2-methyl-2-dichloromethyl-1,3-dithiolane can be reacted with dimercaptoethylsulfide to produce a dimercapto 1,3-dithiolane derivative of the present invention, as shown below.

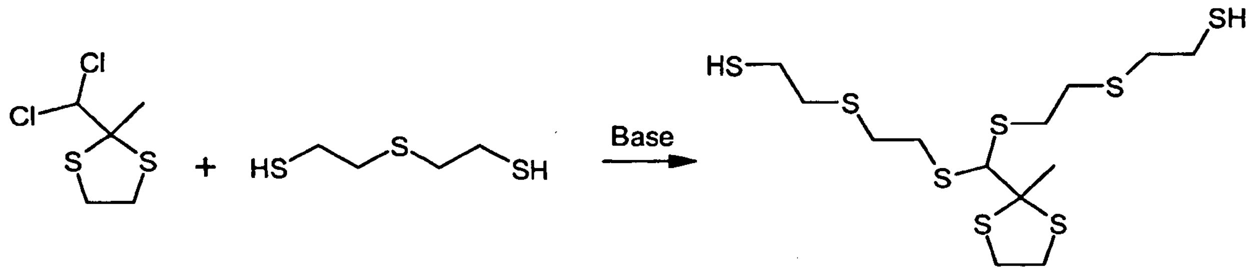


In another non-limiting embodiment, 2-methyl-2-dichloromethyl-1,3-dithiolane can be reacted with 1,2-ethanedithiol to produce a dimercapto 1,3-dithiolane derivative of the present invention, as shown below.

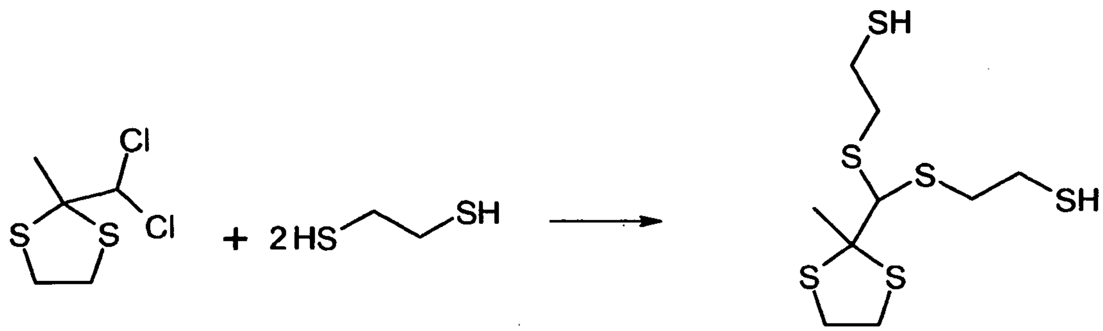

In another non-limiting embodiment, 2-methyl-2-dichloromethyl-1,3-dithiane can be reacted with dimercaptoethylsulfide to produce a dimercapto 1,3-dithiane derivative of the present invention as shown below.

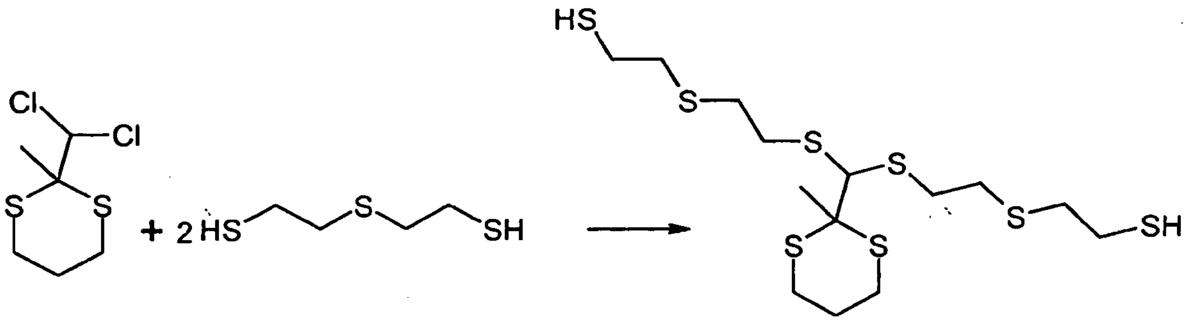

In another non-limiting embodiment, 2-methyl-2-dichloromethyl-1,3-dithiane can be reacted with 1,2-ethanedithiol to produce a dimercapto 1,3-dithiane derivative of the present invention as shown below.

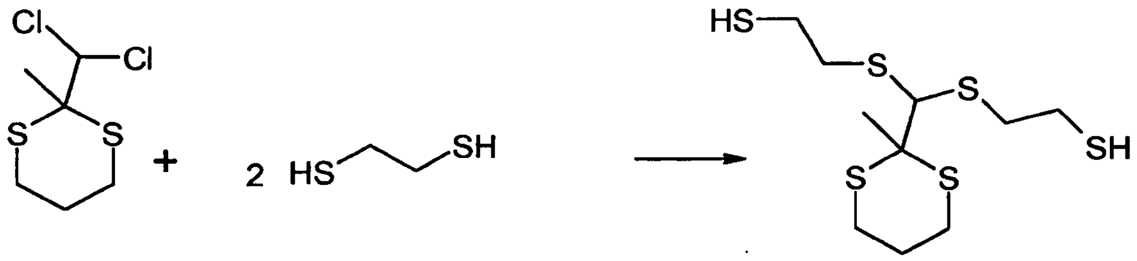

In another non-limiting embodiment, the polythiol for use in the present invention can include at least one oligomeric polythiol prepared by reacting an asym-dichloro derivative with a polymercaptoalkylsulfide as follows.

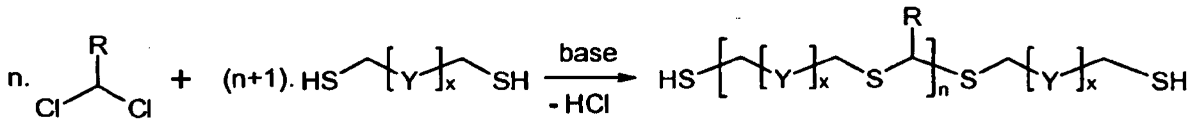


(IV'e)

wherein R can represent $CH_3$, $CH_3CO$, $C_1$ to $C_{10}$ alkyl, cycloalkyl, aryl alkyl, or alkyl-CO; Y can represent $C_1$ to $C_{10}$ alkyl, cycloalkyl, $C_6$ to $C_{14}$ aryl, $(CH_2)_p(S)_m(CH_2)_q$, $(CH_2)_p(Se)_m(CH_2)_q$, $(CH_2)_p(Te)_m(CH_2)_q$ wherein m can be an integer from 1 to 5 and, p and q can each be an integer from 1 to 10; n can be an integer from 1 to 20; and x can be an integer from 0 to 10.

In a further non-limiting embodiment, a polythioether oligomeric dithiol can be prepared by reacting asym-dichloroacetone with polymercaptoalkylsulfide, in the presence of a base. Non-limiting examples of suitable polymercaptoalkylsulfides for use in this reaction can include but are not limited to those materials represented by general formula 2 as previously recited herein. Suitable bases for use in this reaction can include those previously recited herein.

Further non-limiting examples of suitable polymercaptoalkylsulfides for use in the present invention can include branched polymercaptoalkylsulfides. In a non-limiting embodiment, the polymercaptoalkylsulfide can be dimercaptoethylsulfide.

In a non-limiting embodiment, the reaction of asym-dichloro derivative with polymercaptoalkylsulfide can be carried out in the presence of a base. Non-limiting examples of suitable bases can include those previously recited herein.

In another non-limiting embodiment, the reaction of asym-dichloro derivative with polymercaptoalkylsulfide can be carried out in the presence of a phase transfer catalyst. Suitable phase transfer catalysts for use in the present invention are known and varied. Non-limiting examples can include but are not limited to tetraalkylammonium salts and tetraalkylphosphonium salts. In a further non-limiting embodiment, this reaction can be carried out in the presence of tetrabutylphosphonium bromide as phase transfer catalyst. The amount of phase transfer catalyst can vary widely. In a non-limiting embodiment, the amount of phase transfer catalyst can be from 0 to 50 equivalent percent, or from 0 to 10 equivalent percent, or from 0 to 5 equivalent percent, to the polymercaptosulfide reactants.

In another non-limiting embodiment, the preparation of the polythioether oligomeric dithiol can include the use of solvent. Non-limiting examples of suitable solvents can include those previously recited herein.

In a non-limiting embodiment, "n" moles of 1,1-dichloroacetone can be reacted with "n+1" moles of polymercaptoethylsulfide wherein n can represent an integer of from 1 to 20, to produce a polythioether oligomeric dithiol as follows.

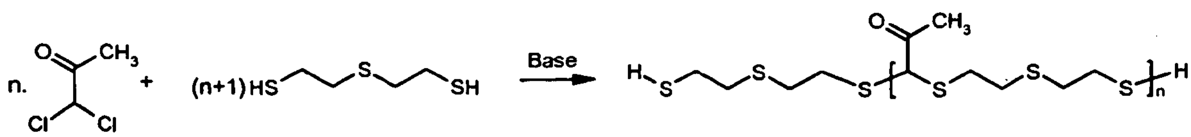


In a further non-limiting embodiment, a polythioether oligomeric dithiol of the present invention can be prepared by introducing "n" moles of 1,1-dichloroethane together with "n+1" moles of polymercaptoethylsulfide as follows,

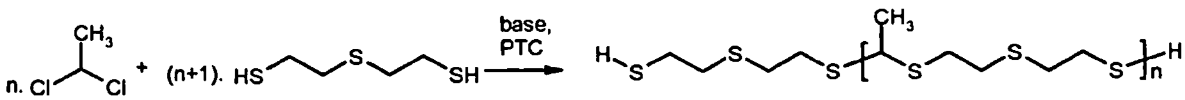


wherein n can represent an integer from 1 to 20.

In alternate non-limiting embodiments, the polythiol for use in the present invention can include at least one oligomeric polythiol represented by the following structural formulas and prepared by the following methods.

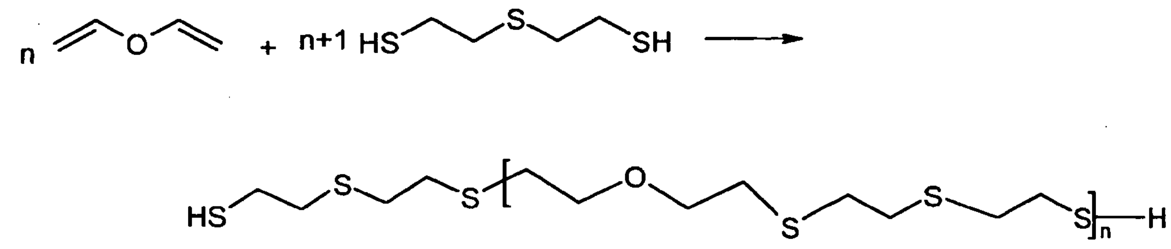

(IV'f)

wherein n can be an integer from 1 to 20.

Various methods of preparing the polythiol of formula (IV'f) are described in detail in United States Patent 6,509,418B1, column 4, line 52 through column 8, line 25. In general, this polythiol can be prepared by reacting reactants comprising one or more polyvinyl ether monomer, and one or more polythiol material. Useful polyvinyl ether monomers can include but are not limited to divinyl ethers represented by structural formula (V'):

$$CH_2=CH\text{-}O\text{-}(\text{--}R^2\text{--}O\text{-})_m\text{ -}CH=CH_2 \qquad (V')$$

wherein $R_2$ can be $C_2$ to $C_6$ n-alkylene, $C_2$ to $C_6$ branched alkylene, $C_6$ to $C_8$ cycloalkylene or $C_6$ to $C_{10}$ alkylcycloalkylene group or $\text{--}[(CH\text{--})_p\text{--}O\text{-}]_q\text{-}(\text{--}CH_2\text{--})_r\text{--}$, and m is a rational number ranging from 0 to 10, p is an independently selected integer ranging from 2 to 6, q is an independently selected integer ranging from 1 to 5 and r is an independently selected integer ranging from 2 to 10.

In a non-limiting embodiment, m can be zero (0).

Non-limiting examples of suitable polyvinyl ether monomers for use can include divinyl ether monomers, such as but not limited to divinyl ether and ethylene glycol divinyl ether.

In alternate non-limiting embodiments, the polyvinyl ether monomer can include from 20 to less than 50 mole percent of the reactants used to prepare the polythiol, or from 30 to less than 50 mole percent.

The divinyl ether of formula (V') can be reacted with a polythiol such as but not limited to a dithiol having the formula (VI'):

HS-R1-SH (VI')

wherein R1 can be a C2 to C6 n-alkylene group; C3 to C6 branched alkylene group, having one or more pendant groups which can include but are not limited to, hydroxyl groups, alkyl groups such as methyl or ethyl groups; alkoxy groups, or C6 to C8 cycloalkylene.

Non-limiting examples of suitable polythiols can include but are not limited to dithiols such as 1,2-ethanedithiol, 1,2-propanedithiol, 1,3-propanedithiol, 1,3-butanedithiol, 1,4-butanedithiol, 2,3-butanedithiol, 1,3-pentanedithiol, 1,5-pentanedithiol, 1,6-hexanedithiol, 1,3-dimercapto-3-methylbutane, dipentenedimercaptan, ethylcyclohexyldithiol (ECHDT), dimercaptodiethylsulfide, methyl-substituted dimercaptodiethylsulfide, dimethyl-substituted dimercaptodiethylsulfide, dimercaptodioxaoctane, 1,5-dimercapto-3-oxapentane and mixtures thereof. In a non-limiting embodiment, the polythiol of formula (VI') can be dimercaptodiethylsulfide (DMDS).

In a non-limiting embodiment, the polythiol material can have a number average molecular weight ranging from 90 to 1000 g/mole, or from 90 to 500 g/mole.

In a further non-limiting embodiment, the stoichiometric ratio of polythiol to divinyl ether materials can be less than one equivalent of polyvinyl ether to one equivalent of polythiol.

In a non-limiting embodiment, the reactants can further include one or more free radical catalysts. Non-limiting examples of suitable free radical catalysts can include azo compounds, such as azobis-nitrile compounds such as but not limited to azo(bis)isobutyronitrile (AIBN); organic peroxides such as but not limited to benzoyl peroxide and t-butyl

peroxide; inorganic peroxides and similar free-radical generators.

In alternate non-limiting embodiments, the reaction can be effected by irradiation with ultraviolet light either with or without a cationic photoinitiating moiety.

In a non-limiting embodiment, the polythiol for use in the present invention can include a material having the following structural formula:

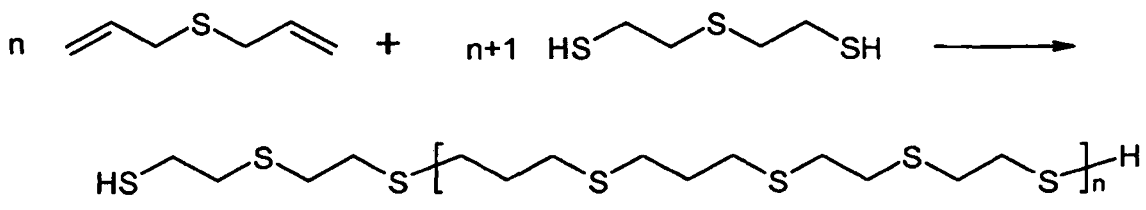

(IV'g)

wherein n can be an integer from 1 to 20.

Various methods of preparing the polythiol of the formula (IV'g) are described in detail in WO 03/042270, page 2, line 16 to page 10, line 7. In general, the polythiol can be a prepolymer having number average molecular weight ranging from 100 to 3000 g/mol, the prepolymer being free from disulfide (-S-S-) linkage, and can be prepared by ultraviolet (UV) catalyzed free radical polymerization in the presence of a suitable photoinitiator. Suitable photoinitiators in usual amounts as known to one skilled in the art can be used for this process. In a non-limiting embodiment, 1-hydroxycyclohexyl phenyl ketone (Irgacure 184) can be used in an amount of from 0.05% to 0.10% by weight, based on the total weight of the polymerizable monomers present in the mixture.

In a non-limiting embodiment, the polythiol of the formula (IV'g) can be prepared by reacting "n" moles of an allyl sulfide and "n+1" moles of dimercaptodiethylsulfide as shown above.

In a non-limiting embodiment, the polythiol for use in the present invention can include a material represented by the following structural formula:

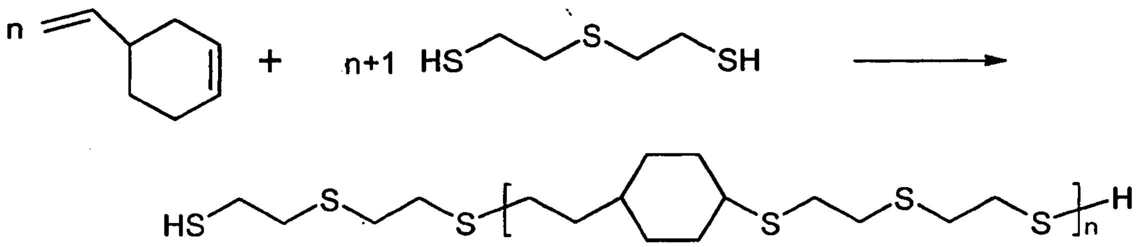

(IV'h)

wherein n can be an integer from 1 to 20.

Various methods for preparing the polythiol of formula (IV'h) are described in detail in WO/01/66623A1, from page 3, line 19 to page 6, line 11. In general, this polythiol can be prepared by the reaction of a thiol such as a dithiol, and an aliphatic, ring-containing nonconjugated diene in the presence of a catalyst. Non-limiting examples of suitable thiols for use in the reaction can include but are not limited to lower alkylene thiols such as ethanedithiol, vinylcyclohexyldithiol, dicyclopentadienedithiol, dipentene dimercaptan, and hexanedithiol; aryl thiols such as benzene dithiol; polyol esters of thioglycolic acid and thiopropionic acid.

Non-limiting examples of suitable cyclodienes can include but are not limited to vinylcyclohexene, dipentene, dicyclopentadiene, cyclododecadiene, cyclooctadiene, 2-cyclopenten-1-yl-ether, 5-vinyl-2-norbornene and norbomadiene.

Non-limiting examples of suitable catalysts for the reaction can include azo or peroxide free radical initiators such as the azobisalkylenenitrile commercially available from DuPont under the trade name VAZO™.

In a further non-limiting embodiment, dimercaptoethylsulfide can be reacted with 4-vinyl-1-cyclohexene, as shown above, with VAZO-52 catalyst.

In another non-limiting embodiment, the polythiol for use in the present invention can include a material represented by the following structural formula:

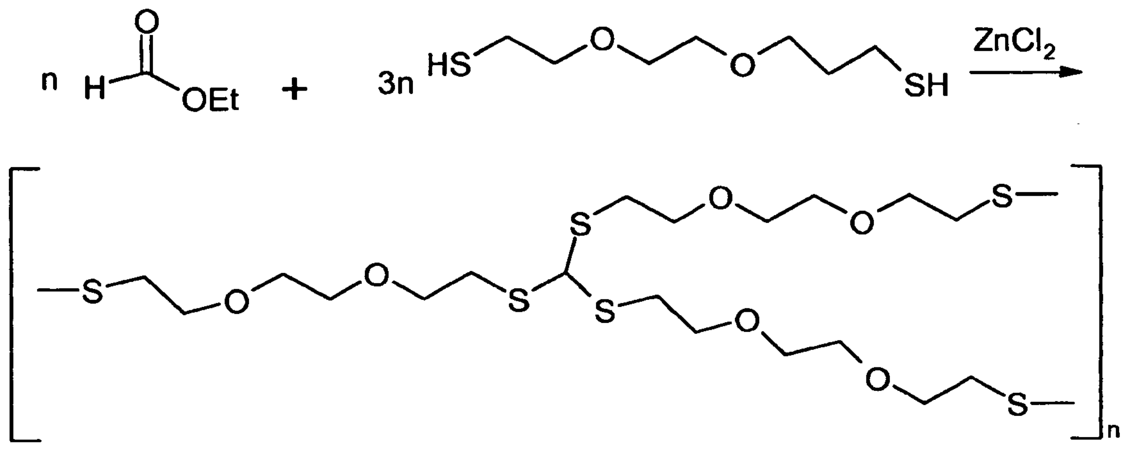


(IV'i)

wherein n can be an integer from 1 to 20.

Various methods of preparing the polythiol of the formula (IV'I) are described in detail in United States Patent 5,225,472, from column 2, line 8 to column 5, line 8.

In a non-limiting embodiment, 1,8-dimercapto-3,6-dioxaooctane (DMDO) can be reacted with ethyl formate, as shown above, in the presence of anhydrous zinc chloride.

In alternate non-limiting embodiments, the active hydrogen-containing material for use in the present invention can be chosen from polyether glycols and polyester glycols having a number average molecular weight of at least 200 g/mol, or at least 300 g/mol, or at least 750 g/mol, or no greater than 1,500 g/mol, or no greater than 2,500 g/mol, or no greater than 4,000 g/mol.

Non-limiting examples of suitable materials having both hydroxyl and thiol groups can include but are not limited to 2-mercaptoethanol, 3-mercapto-1,2-propanediol, glycerin bis(2-mercaptoacetate), glycerin bis(3-mercaptopropionate), 1-hydroxy-4-mercaptocyclohexane, 2,4-dimercaptophenol, 2-mercaptohydroquinone, 4-mercaptophenol, 1,3-dimercapto-2-propanol, 2,3-dimercapto-1-propanol, 1,2-dimercapto-1,3-butanediol, trimethylolpropane bis(2-mercaptoacetate), trimethylolpropane bis(3-mercaptopropionate), pentaerythritol mono(2-mercaptoacetate), pentaerythritol bis(2-mercaptoacetate), pentaerythritol tris(2-mercaptoacetate), pentaerythritol mono(3-mercaptopropionate), pentaerythritol bis(3-mercaptopropionate), pentaerythritol tris(3-mercaptopropionate), hydroxymethyl-tris(mercaptoethylthiomethyl)methane, 1-hydroxyethylthio-3-mercaptoethylthiobenzene, 4-hydroxy-4'-mercaptodiphenylsulfone, dihydroxyethyl sulfide mono(3-mercaptopropionate and hydroxyethylthiomethyl-tris(mercaptoethylthio)methane.

The polycyanate, active hydrogen-containing material and amine-containing curing agent are reacted together in a "one pot" process.

Amine-containing curing agents for use in the present invention are numerous and widely varied. Non-limiting examples of suitable amine-containing curing agents can include but are not limited to aliphatic polyamines, cycloaliphatic polyamines, aromatic polyamines and mixtures thereof. In alternate non-limiting embodiments, the amine-containing curing agent can be a polyamine having at least two functional groups independently chosen from primary amine ($-NH_2$), secondary amine (-NH-) and combinations thereof. In a further non-limiting embodiment, the amine-containing curing agent can have at least two primary amine groups. In another non-limiting embodiment, the amine-containing curing agent can comprise a mixture of a polyamine and at least one material selected from a polythiol and polyol. Non-limiting examples of suitable polythiols and polyols include those previously recited herein. In still another non-limiting embodiment, the amine-containing curing agent can be a sulfur-containing amine-containing curing agent. A non-limiting example of a sulfur-containing amine-containing curing agent can include Ethacure 300 which is commercially available from Albemarle Corporation.

In an embodiment wherein it is desirable to produce a polyureaurethane having low color, the amine-curing agent can be chosen such that it has relatively low color and/or it can be manufactured and/or stored in a manner as to prevent the amine from developing color (e.g., yellow).

Suitable amine-containing curing agents for use in the present invention can include but are not limited to materials having the following chemical formula:

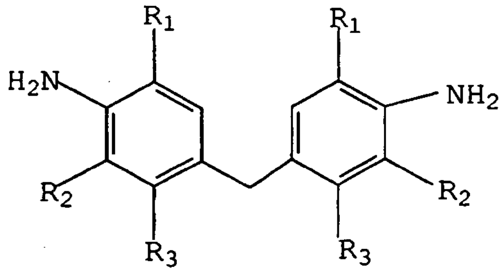

(XII')

wherein $R_1$ and $R_2$ can each be independently chosen from methyl, ethyl, propyl, and isopropyl groups, and $R_3$ can be chosen from hydrogen and chlorine. Non-limiting examples of amine-containing curing agents for use in the present invention include the following compounds, manufactured by Lonza Ltd. (Basel, Switzerland):

LONZACURE.RTM. M-DIPA: $R_1$=$C_3$ $H_7$; $R_2$=$C_3$ $H_7$; $R_3$=H
LONZACURE.RTM. M-DMA: $R_1$=$CH_3$; $R_2$=$CH_3$; $R_3$=H
LONZACURE.RTM. M-MEA: $R_1$=$CH_3$; $R_2$=$C_2$ $H_5$; $R_3$=H
LONZACURE.RTM. M-DEA: $R_1$=$C_2$ $H_5$; $R_2$=$C_2$ $H_5$; $R_3$=H
LONZACURE.RTM. M-MIPA: $R_1$=$CH_3$; $R_2$=$C_3$ $H_7$; $R_3$=H
LONZACURE.RTM. M-CDEA: $R_1$=$C_2$ $H_5$; $R_2$=$C_2$ $H_5$; $R_3$=Cl

wherein $R_1$, $R_2$ and $R_3$ correspond to the aforementioned chemical formula.

In a non-limiting embodiment, the amine-containing curing agent can include but is not limited to a diamine curing agent such as 4,4'-methylenebis(3-chloro-2,6-diethylaniline), (Lonzacure.RTM. M-CDEA), which is available in the United States from Air Products and Chemical, Inc. (Allentown, Pa.). In alternate non-limiting embodiments, the amine-containing curing agent for use in the present invention can include 2,4-diamino-3,5-diethyl-toluene, 2,6-diamino-3,5-diethyl-toluene and mixtures thereof (collectively "diethyltoluenediamine" or "DETDA"), which is commercially available from Albemarle Corporation under the trade name Ethacure 100; dimethylthiotoluenediamine (DMTDA), which is commercially available from Albemarle Corporation under the trade name Ethacure 300; 4,4'-methylene-bis-(2-chloroaniline) which is commercially available from Kingyorker Chemicals under the trade name MOCA. DETDA can be a liquid at room temperature with a viscosity of 156 mPa·s (cPs) at 25°C. DETDA can be isomeric, with the 2,4-isomer range being from 75 to 81 percent while the 2,6-isomer range can be from 18 to 24 percent.

In a non-limiting embodiment, the color stabilized version of Ethacure 100 (i.e., formulation which contains an additive to reduce yellow color), which is available under the name Ethacure 100S may be used in the present invention.

In a non-limiting embodiment, the amine-containing curing agent can act as a catalyst in the polymerization reaction and can be incorporated into the resulting polymerizate.

Non-limiting examples of the amine-containing curing agent can include ethyleneamines. Suitable ethyleneamines can include but are not limited to ethylenediamine (EDA), diethylenetriamine (DETA), triethylenetetramine (TETA), tetraethylenepentamine (TEPA), pentaethylenehexamine (PEHA), piperazine, morpholine, substituted morpholine, piperidine, substituted piperidine, diethylenediamine (DEDA), and 2-amino-1-ethylpiperazine. In alternate non-limiting embodiments, the amine-containing curing agent can be chosen from one or more isomers of $C_1$-$C_3$ dialkyl toluenediamine, such as but not limited to 3,5-dimethyl-2,4-toluenediamine, 3,5-dimethyl-2,6-toluenediamine, 3,5-diethyl-2,4-toluenediamine, 3,5-diethyl-2,6-toluenediamine, 3,5-diisopropyl-2,4-toluenediamine, 3,5-diisopropyl-2,6-toluenediamine, and mixtures thereof. In alternate non-limiting embodiments, the amine-containing curing agent can be methylene dianiline or trimethyleneglycol di(para-aminobenzoate).

In alternate non-limiting embodiments of the present invention, the amine-containing curing agent can include one of the following general structures:

(XIII)

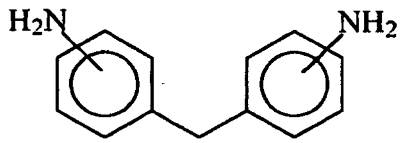

(XIV) 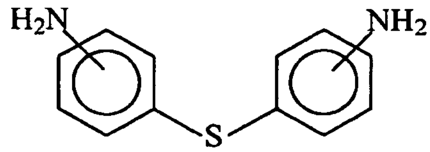

(XV) 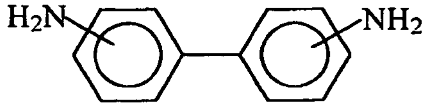

In further alternate non-limiting embodiments, the amine-containing curing agent can include one or more methylene bis anilines which can be represented by the general formulas XVI-XX, one or more aniline sulfides which can be represented by the general formulas XXI-XXV, and/or one or more bianilines which can be represented by the general formulas XXVI-XXVIX,

(XVI) 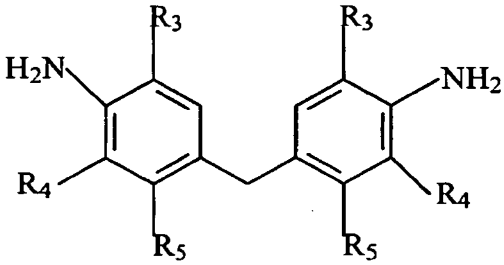

(XVII) 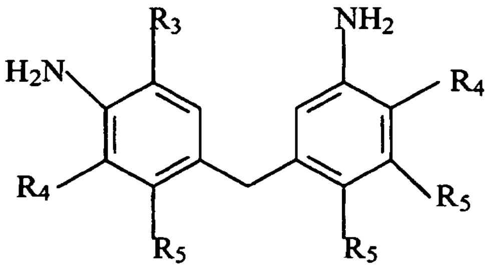

(XVIII) 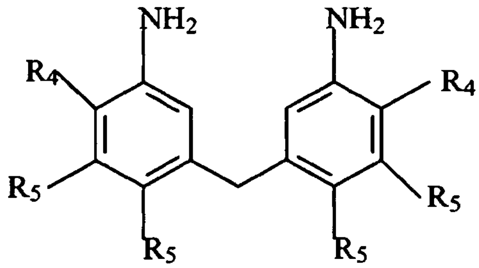

(XIX) 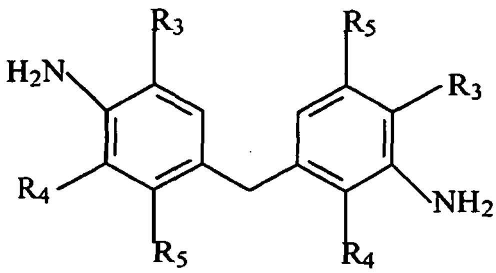

(XX)

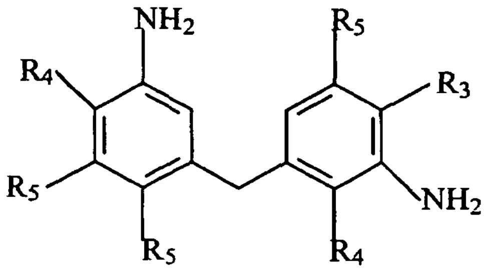

(XXI)

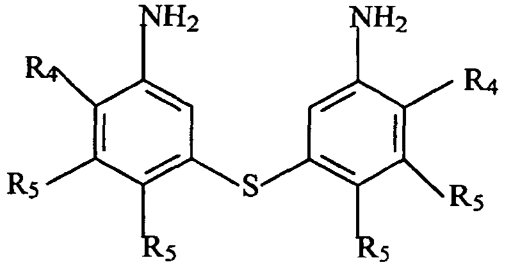

(XXII)

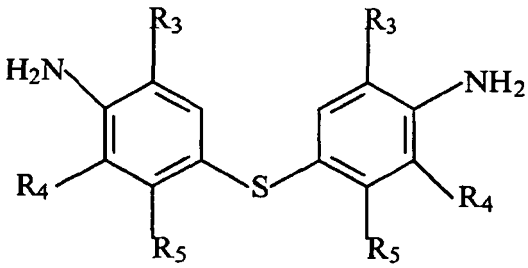

(XXIII)

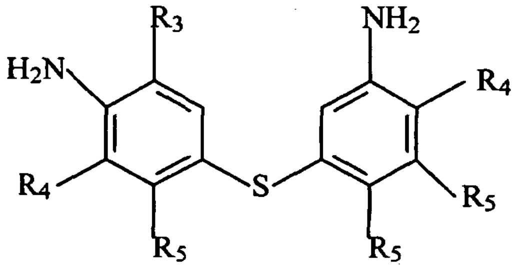

(XXIV)

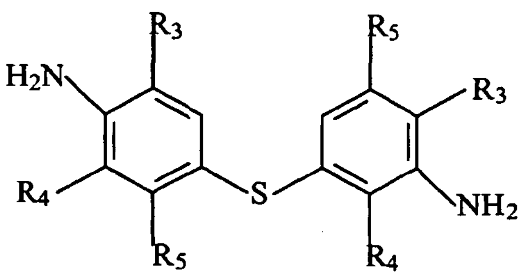

(XXV)

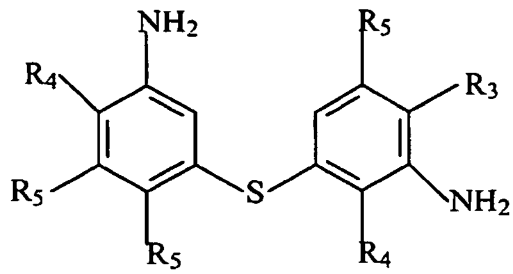

(XXVI)

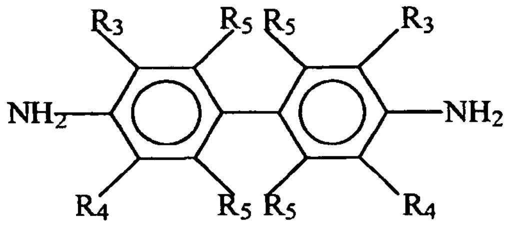

(XXVII)

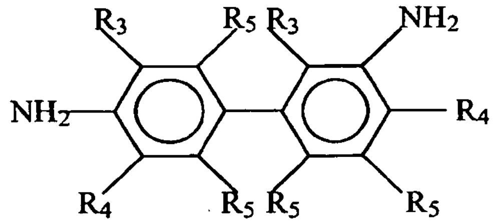

(XXVIII)

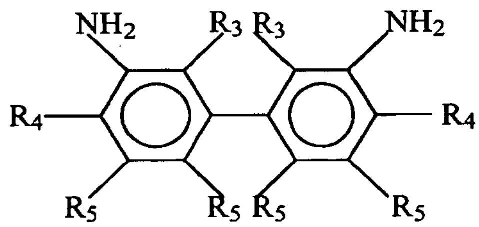

(XXIX)

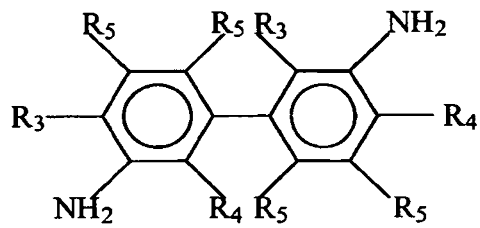

wherein $R_3$ and $R_4$ can each independently represent $C_1$ to $C_3$ alkyl, and $R_5$ can be chosen from hydrogen and halogen, such as but not limited to chlorine and bromine. The diamine represented by general formula XV can be described generally as a 4,4'-methylene-bis(dialkylaniline). Suitable non-limiting examples of diamines which can be represented by general formula XV include but are not limited to 4,4'-methylene-bis(2,6-dimethylaniline), 4,4'-methylene-bis(2,6-diethylaniline), 4,4'-methylene-bis(2-ethyl-6-methylaniline), 4,4'-methylene-bis(2,6-diisopropylaniline), 4,4'-methylene-bis(2-isopropyl-6-methylaniline) and 4,4'-methylene-bis(2,6-diethyl-3-chloroaniline).

In a further non-limiting embodiment, the amine-containing curing agent can include materials which can be represented by the following general structure (XXX):

(XXX) 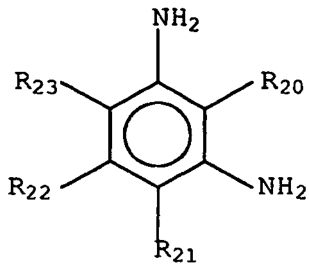

where $R_{20}$, $R_{21}$, $R_{22}$, and $R_{23}$ can be independently chosen from H, $C_1$ to $C_3$ alkyl, $CH_3$-S- and halogen, such as but not limited to chlorine or bromine. In a non-limiting embodiment of the present invention, the amine-containing curing agent which can be represented by general formula XXX can include diethyl toluene diamine (DETDA) wherein $R_{23}$ is methyl, $R_{20}$ and $R_{21}$ are each ethyl and $R_{22}$ is hydrogen. In a further non-limiting embodiment, the amine-containing curing agent can include 4,4'-methylenedianiline.

In another non-limiting embodiment the sulfur-containing polyureaurethane can be polymerized by intoducing together the polycyanate, active hydrogen-containing material, and amine-containing curing agent, and degassing the mixture. In a further non-limiting embodiment, this mixture can be degassed under vacuum.

In another non-limiting embodiment, wherein a lens can be formed, the degassed mixture can be introduced into a mold and the mold can be heated using a variety of conventional techniques known in the art. The thermal cure cycle can vary depending on, for example, the reactivity and molar ratio of the reactants and the presence of catalyst(s). In a non-limiting embodiment, the thermal cure cycle can include heating the polycyanate, active-hydrogen-containing material and curing agent mixture, from room temperature to 200°C over a period of from 0.5 hours to 72 hours.

In a non-limiting embodiment, a urethane-forming catalyst can be used in the present invention to enhance the reaction of the polyurethane-forming materials. Suitable urethane-forming catalysts can vary, for example, suitable urethane-forming catalysts can include those catalysts that are useful for the formation of urethane by reaction of the NCO and OH-containing materials, and which have little tendency to accelerate side reactions leading to allophonate and isocyanate formation. Non-limiting examples of suitable catalysts can be chosen from the group of Lewis bases, Lewis acids and insertion catalysts as described in Ullmann's Encyclopedia of Industrial Chemistry, 5th Edition, 1992, Volume A21, pp. 673 to 674. In a non-limiting embodiment, the catalyst can be a stannous salt of an organic acid, such as but not limited to stannous octoate, dibutyl tin dilaurate, dibutyl tin diacetate, dibutyl tin mercaptide, dibutyl tin dimaleate, dimethyl tin diacetate, dimethyl tin dilaurate, 1,4-diazabicyclo[2.2.2]octane, and mixtures thereof. In alternate non-limiting embodiments, the catalyst can be zinc octoate, bismuth, or ferric acetylacetonate.

Further non-limiting examples of suitable catalysts can include tertiary amines such as but not limited to triethylamine, triisopropylamine and N,N-dimethylbenzylamine. Such suitable tertiary amines are disclosed in United States Patent 5,693,738 at column 10, lines 6-38.

In a non-limiting embodiment the catalyst can be chosen from phosphines, tertiary ammonium salts and tertiary amines, such as but not limited to triethylamine; triisopropylamine and N,N-dimethylbenzylamine. Additional non-limiting examples of suitable tertiary amines are disclosed in United States Patent 5,693,738 at column 10 lines 6 through 38.

In alternate non-limiting embodiments, the sulfur-containing polyureaurethane of the present invention can have a equivalent ratio of ($-NH_2$ + -NH- + -OH + SH) to (NCO + NCS) of at least 0.4:1, or at least 0.8:1, or 1.0:1, or 2:0:1.0 or less.

In a non-limiting embodiment, the polyether-containing polyol can comprise block polymers including blocks of ethylene oxide-propylene oxide and/or ethylene oxide-butylene oxide. In a non-limiting embodiment, the polyether-containing polyol can comprise a block polymer of the following chemical formula:

(XXXI') $$H-(O-CRRCRR-Y_n)_a-(CRRCRR-Y_n-O)_b-(CRRCRR-Y_n-O)_c-H$$

wherein R can represent hydrogen or $C_1$-$C_6$ alkyl; Y can represent $CH_2$; n can be an integer from 0 to 6; a, b, and c can each be an integer from 0 to 300, wherein a, b and c are chosen such that the number average molecular weight of the polyol does not exceed 32,000 g/mol.

In alternate non-limiting embodiments, various known additives can be incorporated into the sutfur-containing polyureaurethane of the present invention. Such additives can include but are not limited to light stabilizers, heat stabilizers, antioxidants, ultraviolet light absorbers, mold release agents, static (non-photochromic) dyes, pigments and flexibilizing additives, such as but not limited to alkoxylated phenol benzoates and poly(alkylene glycol) dibenzoates. Non-limiting examples of anti-yellowing additives can include 3-methyl-2-butenol, organo pyrocarbonates and triphenyl phosphite (CAS registry no. 101-02-0). Such additives can be present in an amount such that the additive constitutes less than 10 percent by weight, or less than 5 percent by weight, or less than 3 percent by weight, based on the total weight

of the prepolymer. In alternate non-limiting embodiments, the aforementioned optional additives can be mixed with the polycyanate. In a further embodiment, the optional additives can be mixed with hydrogen-containing material.

In a non-limiting embodiment, the resulting sulfur-containing polyureaurethane of the present invention when at least partially cured can be solid, and essentially transparent such that it is suitable for optical or ophthalmic applications. In alternate non-limiting embodiments, the sulfur-containing polyureaurethane can have a refractive index of at least 1.57, or at least 1.58, or at least 1.60, or at least 1.62. In further alternate non-limiting embodiments, the sulfur-containing polyureaurethane can have an Abbe number of at least 32, or at least 35, or at least 38, or at least 39, or at least 40, or at least 44.

In a non-limiting embodiment, the sulfur-containing polyureaurethane when polymerized and at least partially cured can demonstrate good impact resistance/strength. Impact resistance can be measured using a variety of conventional methods known to one skilled in the art. In a non-limiting embodiment, the impact resistance is measured using the Impact Energy Test which consists of testing a flat sheet of polymerizate having a thickness of 3mm, by dropping various balls of increasing weight from a distance of 50 inches (1.25 meters) onto the center of the sheet. If the sheet is determined to have failed the test when the sheet fractures. As used herein, the term "fracture" refers to a crack through the entire thickness of the sheet into two or more separate pieces, or detachment of one or more pieces from the backside of the sheet (i.e., the side of the sheet opposite the side of impact). In this embodiment, using the Impact Energy Test as described herein, the impact strength can be at least 2.0 joules, or at least 4.95 joules.

Further, the sulfur-containing polyureaurethane of the present invention when at least partially cured can have low density. In a non-limiting embodiment, the density can be from greater than 1.0 to less than 1.25 g/cm$^3$, or from greater than 1.0 to less than 1.3 g/cm$^3$. In a non-limiting embodiment, the density is measured using a DensiTECH instrument manufactured by Tech Pro, Incorporated. In a further non-limiting embodiment, the density is measured in accordance with ASTM D297.

Solid articles that can be prepared using the sulfur-containing polyureaurethane of the present invention include but are not limited to optical lenses, such as plano and ophthalmic lenses, sun lenses, windows, automotive transparencies, such as windshields, sidelights and backlights, and aircraft transparencies.

In a non-limiting embodiment, the sulfur-containing polyureaurethane polymerizate of the present invention can be used to prepare photochromic articles, such as lenses. In a further embodiment, the polymerizate can be transparent to that portion of the electromagnetic spectrum which activates the photochromic substance(s), i.e., that wavelength of ultraviolet (UV) light that produces the colored or open form of the photochromic substance and that portion of the visible spectrum that includes the absorption maximum wavelength of the photochromic substance in its UV activated form, i.e., the open form.

A wide variety of photochromic substances can be used in the present invention. In a non-limiting embodiment, organic photochromic compounds or substances can be used. In alternate non-limiting embodiments, the photochromic substance can be incorporated, e.g., dissolved, dispersed or diffused into the polymerizate, or applied as a coating thereto.

In a non-limiting embodiment, the organic photochromic substance can have an activated absorption maximum within the visible range of greater than 590 nanometers. In a further non-limiting embodiment, the activated absorption maximum within the visible range can be between greater than 590 to 700 nanometers. These materials can exhibit a blue, bluish-green, or bluish-purple color when exposed to ultraviolet light in an appropriate solvent or matrix. Non-limiting examples of such substances that are useful in the present invention include but are not limited to spiro(indoline)naphthoxazines and spiro(indoline)benzoxazines. These and other suitable photochromic substances are described in U.S. Patents: 3,562,172; 3,578,602; 4,215,010; 4,342,668; 5,405,958; 4,637,698; 4,931,219; 4,816,584; 4,880,667; 4,818,096.

In another non-limiting embodiment, the organic photochromic substances can have at least one absorption maximum within the visible range of between 400 and less than 500 nanometers. In a further non-limiting embodiment, the substance can have two absorption maxima within this visible range. These materials can exhibit a yellow-orange color when exposed to ultraviolet light in an appropriate solvent or matrix. Non-limiting examples of such materials can include certain chromenes, such as but not limited to benzopyrans and naphthopyrans. Many of such chromenes are described in U.S. Patents 3,567,605; 4,826,977; 5,066,818; 4,826,977; 5,066,818; 5,466,398; 5,384,077; 5,238,931; and 5,274,132.

In another non-limiting embodiment, the photochromic substance can have an absorption maximum within the visible range of between 400 to 500 nanometers and an absorption maximum within the visible range of between 500 to 700 nanometers. These materials can exhibit color(s) ranging from yellow/brown to purple/gray when exposed to ultraviolet light in an appropriate solvent or matrix. Non-limiting examples of these substances can include certain benzopyran compounds having substituents at the 2-position of the pyran ring and a substituted or unsubstituted heterocyclic ring, such as a benzothieno or benzofurano ring fused to the benzene portion of the benzopyran. Further non-limiting examples of such materials are disclosed in U.S. Patent No. 5,429,774.

In a non-limiting embodiment, the photochromic substance for use in the present invention can include photochromic organo-metal dithizonates, such as but not limited to (arylazo)-thioformic arylhydrazidates, such as but not

limited to mercury dithizonates which are described, for example, in U.S. Patent 3,361,706. Fulgides and fulgimides, such as but not limited to 3-furyl and 3-thienyl fulgides and fulgimides which are described in U.S. Patent 4,931,220 at column 20, line 5 through column 21, line 38, can be used in the present invention.

In alternate non-limiting embodiments, the photochromic articles of the present invention can include one photochromic substance or a mixture of more than one photochromic substances. In further alternate non-limiting embodiment, various mixtures of photochromic substances can be used to attain activated colors such as a near neutral gray or brown.

The amount of photochromic substance employed can vary. In alternate non-limiting embodiments, the amount of photochromic substance and the ratio of substances (for example, when mixtures are used) can be such that the polymerizate to which the substance is applied or in which it is incorporated exhibits a desired resultant color, e.g., a substantially neutral color such as shades of gray or brown when activated with unfiltered sunlight, i.e., as near a neutral color as possible given the colors of the activated photochromic substances. In a non-limiting embodiment, the amount of photochromic substance used can depend upon the intensity of the color of the activated species and the ultimate color desired.

In alternate non-limiting embodiments, the photochromic substance can be applied to or incorporated into the polymerizate by various methods known in the art. In a non-limiting embodiment, the photochromic substance can be dissolved or dispersed within the polymerizate. In a further non-limiting embodiment, the photochromic substance can be imbibed into the polymerizate by methods known in the art. The term "imbibition" or "imbibe" includes permeation of the photochromic substance alone into the polymerizate, solvent assisted transfer absorption of the photochromic substance into a porous polymer, vapor phase transfer, and other such transfer mechanisms. In a non-limiting embodiment, the imbibing method can include coating the photochromic article with the photochromic substance; heating the surface of the photochromic article; and removing the residual coating from the surface of the photochromic article. In alternate non-limiting embodiments, the imbibtion process can include immersing the polymerizate in a hot solution of the photochromic substance or by thermal transfer.

In alternate non-limiting embodiments, the photochromic substance can be a separate layer between adjacent layers of the polymerizate, e.g., as a part of a polymer film; or the photochromic substance can be applied as a coating or as part of a coating placed on the surface of the polymerizate.

The amount of photochromic substance or composition containing the same applied to or incorporated into the polymerizate can vary. In a non-limiting embodiment, the amount can be such that a photochromic effect discernible to the naked eye upon activation is produced. Such an amount can be described in general as a photochromic amount. In alternate non-limiting embodiments, the amount used can depend upon the intensity of color desired upon irradiation thereof and the method used to incorporate or apply the photochromic substance. In general, the more photochromic substance applied or incorporated, the greater the color intensity. In a non-limiting embodiment, the amount of photochromic substance incorporated into or applied onto a photochromic optical polymerizate can be from 0.15 to 0.35 millig per square centimeter of surface to which the photochromic substance is incorporated or applied.

In another embodiment, the photochromic substance can be added to the sulfur-containing polyureaurethane prior to polymerizing and/or cast curing the material. In this embodiment, the photochromic substance used can be chosen such that it is resistant to potentially adverse interactions with, for example, the isocyanate, isothiocyante and amine groups present. Such adverse interactions can result in deactivation of the photochromic substance, for example, by trapping them in either an open or closed form.

Further non-limiting examples of suitable photochromic substances for use in the present invention can include photochromic pigments and organic photochromic substances encapsulated in metal oxides such as those disclosed in U.S. Patents 4,166,043 and 4,367,170; organic photochromic substances encapsulated in an organic polymerizate such as those disclosed in U.S. Patent 4,931,220.

## EXAMPLES

In the following examples, unless otherwise stated, the 1H NMR and 13C NMR were measured on a Varian Unity Plus (200 MHz) machine; the Mass Spectra were measured on a Mariner Bio Systems apparatus; the refractive index and Abbe number were measured on a multiple wavelength Abbe Refractometer Model DR-M2 manufactured by ATAGO Co., Ltd.; the refractive index and Abbe number of liquids were measured in accordance with ASTM-D1218; the refractive index and Abbe number of solids was measured in accordance with ASTM-D542; the density of solids was measured in accordance with ASTM-D792; and the viscosity was measured using a Brookfield CAP 2000+ Viscometer.

The SH groups within the product were determined using the following procedure. A sample size (0.1 mg) of the product was combined with 50 mL of tetrahydrofuran (THF)/propylene glycol (80/20) and stirred at room temperature until the sample was substantially dissolved. While stirring, 25.0 mL of 0.1 N iodine solution (which was commercially obtained from Aldrich 31, 8898-1) was added to the mixture and then allowed to react for a time period of from 5 to 10

minutes. To this mixture was added 2.0 mL concentrated HCl. The mixture was then titrated potentiometrically with 0.1 N sodium thiosulfate in the millivolt (mV) mode. A blank value was initially obtained by titrating 25.0 mL of iodine (including 1 mL of concentrated hydrochloric acid) with sodium thiosulfate in the same manner as conducted with the product sample.

$$\%SH = \frac{\text{mLsBlank} - \text{mLSSample})(\text{Normality } Na2S2O3)(3.307)}{\text{sample weight, g}}$$

REFERENCE EXAMPLE 1 - Synthesis of PTE Dithiol 6 (DMDS/VCH, 1:2 mole ratio)

Charged into a 1-liter 4-necked flask equipped with a mechanical stirrer, thermometer and two gas passing adapters (one for inlet and one for outlet), 2-dimercaptodiethyl sulfide (DMDS) (888.53g, 5.758 moles). The flask was flushed with dry nitrogen and 4-vinyl-1-cyclohexene (VCH) (311.47g, 2.879 moles) was added with stirring during a time period of 2hr, 15 min. The reaction temperature increased from room temperature to 62°C after 1 hr of addition. Following addition of the vinylcyclohexene, the reaction temperature was 37°C. The reaction mixture was then heated to a temperature of 60°C, and five 0.25g-portions of free radical initiator Vazo-52 (2,2'-azobis(2,4-dimethylpentanenitrile) obtained from DuPont), were added. Each portion was added after interval of one hour. The reaction mixture was evacuated at 60°C/533-667 kPa (4-5mm Hg) for one hour to give 1.2 kg (yield: 100%) of a colorless liquid, with the following properties:

Viscosity 300 mPa·s (cps) @ 25°C (Brookfield CAP 2000+, spindle #3, 500 rpm); refractive index (e-line) = 1.597 (20°C); Abbe Number = 39; SH groups content 16.7%.

EXAMPLE 2 - One pot synthesis of polythiourea/urethane material

4,4-dicyclohexylmethane diisocyanate (Desmodur W) from Bayer (42.00g, 0.16 mole) was degassed under vacuum at room temperature for two hours. PTE Dithiol 6 (32.00g, 0.08 mole), diethyltoluenediamine (DETDA) (11.40 g, 0.064 mole) and DMDS (2.46 g, 0.016 mole) were mixed and degassed under vacuum at room temperature for two hours. The two mixtures were then mixed at the same temperature and charged between preheated glass plates mold. The material was cured in a preheated oven at a temperature of 130°C for 24 hours. The cured material was clear. The results were as follows: refractive index (e-line) 1.582 (20°C), and Abbe number 40.

## Claims

1. A sulfur-containing polyureaurethane adapted to have a refractive index of at least 1.57 determined by ASTM D 542-00, an Abbe number of at least 32 determined by ASTM D 542-00 and a density of less than 1.3 g/cm$^3$, when at least partially cured, that is prepared by reacting together

   (a) a polyisocyanate selected from aliphatic diisocyanates, cycloaliphatic diisocyanates, aromatic diisocyanates, cyclic dimers and cyclic trimers thereof, and mixtures thereof;
   (b) a sulfur-containing active hydrogen material; and
   (c) an amine-containing curing agent

   in a "one pot" process.

2. A sulfur-containing polyureaurethane adapted to have a refractive index of at least 1.57 determined by ASTM D 542-00, an Abbe number of at least 32 determined by ASTM D 542-00 and a density of less than 1.3 g/cm$^3$, when at least partially cured, that is prepared by reacting together

   (a) a polyisothiocyanate;
   (b) an active hydrogen-containing material selected from hydroxyl-containing materials, SH-containing materials, and materials having both hydroxyl and thiol functional groups; and
   (c) an amine-containing curing agent

   in a "one pot" process.

3. The sulfur-containing polyureaurethane of claim 1 wherein said amine-containing curing agent is a sulfur-containing amine-containing curing agent.

**4.** The sulfur-containing polyureaurethane of claim 1 wherein said amine-containing curing agent is a mixture of amine-containing curing agent and at least one material chosen from polythiol and polyol.

**5.** The sulfur-containing polyureaurethane of claim 1 wherein the sulfur-containing active hydrogen material is a SH-containing material, preferably a polythiol.

**6.** The sulfur-containing polyureaurethane of claim 1 wherein the sulfur-containing active hydrogen material is a mixture of polythiol and polyol free of sulfur.

**7.** The sulfur-containing polyureaurethane of claim 5 wherein said polythiol is chosen from aliphatic polythiols, cycloaliphatic polythiols, aromatic polythiols, polymeric polythiols, polythiols containing ether linkages, polythiols containing one or more sulfide linkages.

**8.** The sulfur-containing polyureaurethane of claim 5 wherein the polythiol comprises at least one of the following materials:

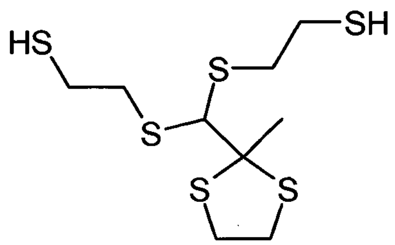

(IV'a)

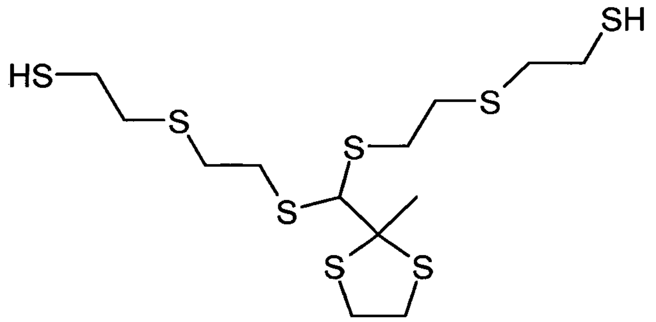

(IV'b)

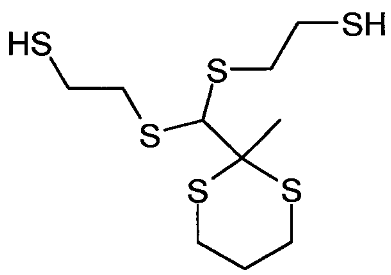

(IV'c)

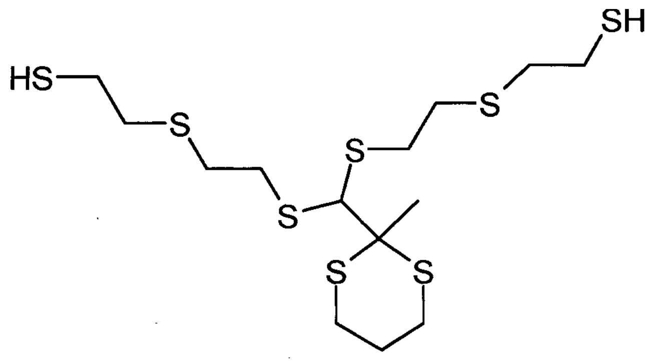

(IV'd)

**9.** The sulfur-containing polyureaurethane of claim 5 wherein the polythiol comprises at least one material represented by the following structural formula:

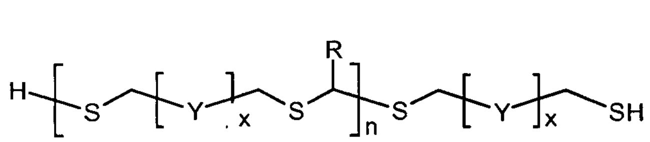

wherein R can represent $CH_3$, $CH_3CO$, $C_1$ to $C_{10}$ alkyl, cycloalkyl, aryl alkyl, or alkyl-CO; Y can represent $C_1$ to $C_{10}$ alkyl, cycloalkyl, $C_6$ to $C_{14}$ aryl, $(CH_2)_p(S)_m(CH_2)_q$, $(CH_2)_p(Se)_m(CH_2)_q$, $(CH_2)_p(Te)_m(CH_2)_q$ wherein m can be an integer from 1 to 5 and, p and q can each be an integer from 1 to 10; n can be an integer from 1 to 20; and x can be an integer from 0 to 10.

**10.** The sulfur-containing polyureaurethane of claim 5 wherein the polythiol comprises at least one of the following materials:

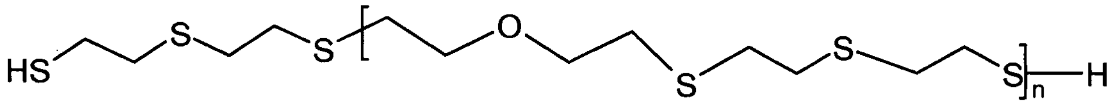

(IV'f)

n

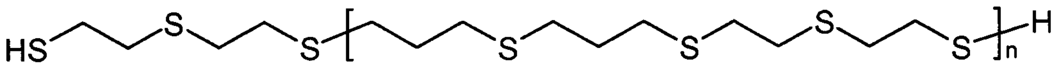

(IV'g)

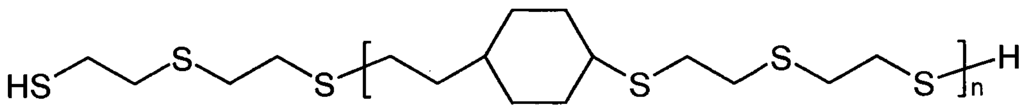

(IV'h)

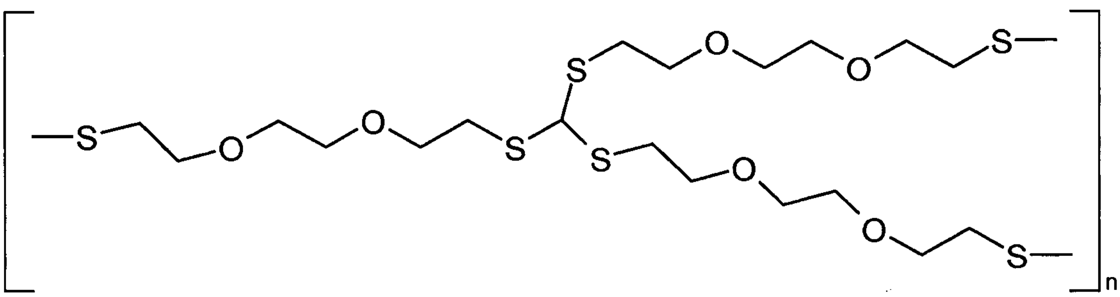

(IV'i)

wherein n can be an integer from 1 to 20.

**11.** The sulfur-containing polyureaurethane of claim 1 wherein the sulfur-containing active hydrogen material is a hydroxyl functional polysulfide, preferably a hydroxyl functional polysulfide further comprising SH-functionality.

**12.** A method of preparing a polyureaurethane of claims 1 or 2 comprising reacting the polyisocyanate (a), the active hydrogen material (b); and the amine-containing curing agent (c) in a one-pot process.

**13.** An optical article, preferably an ophthalmic lens, comprising the sulfur-containing polyureaurethane of any of claims 1 to 11.

**14.** A photochromic article comprising the sulfur-containing polyureaurethane of any of claims 1 to 11.

## Patentansprüche

1. Schwefelhaltiges Polyharnstoffurethan, das angepasst ist, um einen Brechungsindex von 1,57, bestimmt nach ASTM D542-00, eine Abbe-Nummer von mindestens 52, bestimmt nach ASTM D542-00, und eine Dichte von weniger als 1,3 g pro $cm^3$ aufzuweisen, wenn es mindestens teilweise gehärtet ist, das hergestellt ist durch Reaktion von

   (a) einem Polyisocyanat, ausgewählt aus aliphatischen Diisocyanaten, cycloaliphatischen Diisocyanaten, aromatischen Diisocyanaten, cyclischen Dimeren und cyclischen Trimeren davon und Mischungen derselben,
   (b) einem schwefelhaltigen aktiven Wasserstoff enthaltenden Material und
   (c) einem aminhaltigen Härtungsmittel in einem "Ein-Topf"-Verfahren.

2. Schwefelhaltiges Polyharnstoffurethan, das angepasst ist, um einen Brechungsindex von 1,57, bestimmt nach ASTM D542-00, eine Abbe-Nummer von mindestens 32, bestimmt nach ASTM D542-00, und eine Dichte von weniger als 1,3 g/$cm^3$ aufzuweisen, wenn es mindestens teilweise gehärtet ist, das hergestellt ist durch Reaktion von

   (a) einem Polyisothiocyanat,
   (b) einem aktiven Wasserstoff enthaltenden Material, ausgewählt aus hydroxylhaltigen Materialien, SH-haltigen Materialien und Materialien mit sowohl hydroxyl- als auch thiolfunktionellen Gruppen und
   (c) einem aminhaltigen Härtungsmittel in einem "Ein-Topf"-Verfahren.

3. Schwefelhaltiges Polyharnstoffurethan nach Anspruch 1, wobei dieses aminhaltige Härtungsmittel ein schwefelhaltiges aminhaltiges Härtungsmittel ist.

4. Schwefelhaltiges Polyharnstoffurethan nach Anspruch 1, wobei dieses aminhaltige Härtungsmittel eine Mischung aus aminhaltigem Härtungsmittel und mindestens einem Material ausgewählt aus Polythiol und Polyol ist.

5. Schwefelhaltiges Polyharnstoffurethan nach Anspruch 1, wobei das schwefelhaltige aktiven Wasserstoff enthaltende Material ein SH-haltiges Material, vorzugsweise ein Polythiol, ist.

6. Schwefelhaltiges Polyharnstoffurethan nach Anspruch 1, wobei das schwefelhaltige aktiven Wasserstoff enthaltende Material eine Mischung aus Polythiol und schwefelfreiem Polyol ist.

7. Schwefelhaltiges Polyharnstoffurethan nach Anspruch 5, wobei dieses Polythiol aus aliphatischen Polythiolen, cycloaliphatischen Polythiolen, aromatischen Polythiolen, polymeren Polythiolen, Polythiolen mit Etherverknüpfungen, Polythiolen, die eine oder mehrere Sulfidverknüpfungen enthalten, ausgewählt ist.

8. Schwefelhaltiges Polyharnstoffurethan nach Anspruch 5, wobei das Polythiol mindestens eines der folgenden Materialien enthält:

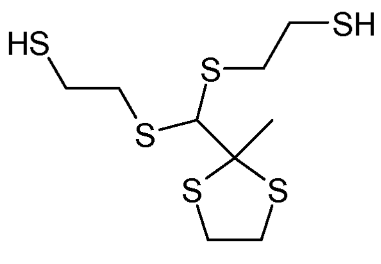
(IV'a)

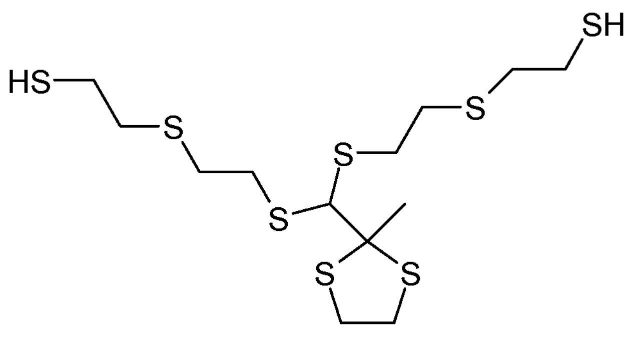
(IV'b)

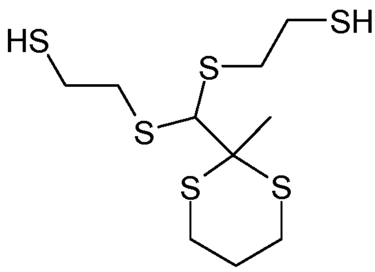

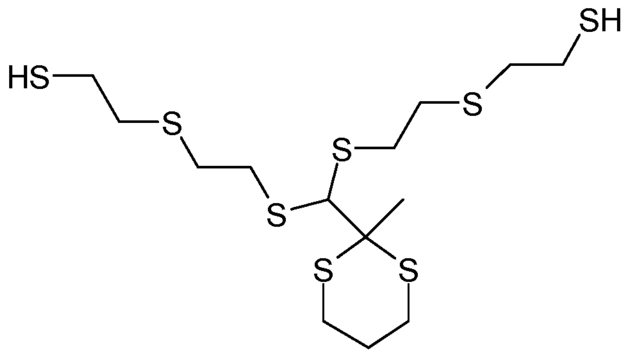

(IV'c) (IV'd)

**9.** Schwefelhaltiges Polyharnstoffurethan nach Anspruch 5, wobei das Polythiol mindestens ein Material enthält, das durch die folgende Strukturformel dargestellt ist:

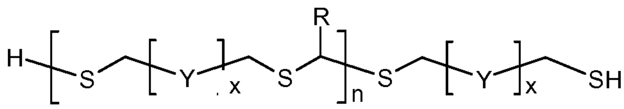

worin R durch $CH_3$, $CH_3CO$, $C_1$- bis $C_{10}$ Alkyl, Cycloalkyl, Arylalkyl oder Alkyl-CO wiedergegeben sein kann, Y durch $C_1$- bis $C_{10}$-Alkyl, Cycloalkyl, $C_6$- bis $C_{14}$-Aryl, $(CH_2)_p(S)_m(CH_2)_q$, $(CH_2)_p(Se)_m(CH_2)_q$, $(CH_2)_p(Te)_m(CH_2)_q$ wiedergegeben sein kann, worin m eine ganze Zahl von 1 bis 5 und, p und q jeweils eine ganze Zahl von 1 bis 10 sein können; n eine ganze Zahl von 1 bis 20 sein kann und x eine ganze Zahl von 0 bis 10 sein kann,.

**10.** Schwefelhaltiges Polyharnstoffurethan nach Anspruch 5, wobei das Polythiol mindestens eines der folgenden Materialien enthält:

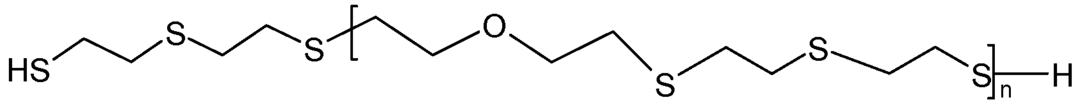

(IV'f)

n

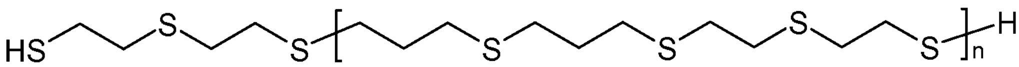

(IV'g)

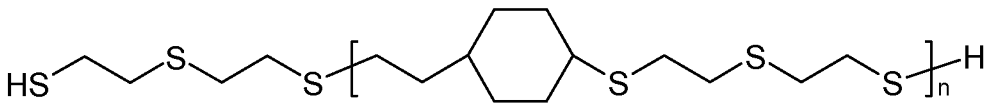

(IV'h)

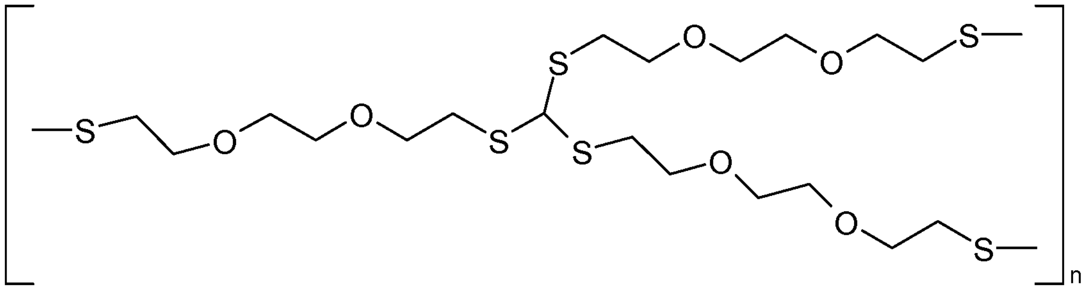

(IV'i)

worin n eine ganze Zahl von 1 bis 20 sein kann.

**11.** Schwefelhaltiges Polyharnstoffurethan nach Anspruch 1, wobei das schwefelhaltige aktiven Wasserstoff enthaltende Material ein hydroxylfunktionelles Polysulfid, vorzugsweise ein hydroxylfunktionelles Polysulfid, das ferner SH-Funktionalität aufweist, ist.

**12.** Verfahren zur Herstellung eines Polyharnstoffurethans nach Anspruch 1 oder 2, umfassend Umsetzen des Polyisocyanats (a), des aktiven Wasserstoff enthaltenden Materials (b) und des aminhaltigen Härtungsmittels (c) in einem "Ein-Topf"-Verfahren.

**13.** Optischer Gegenstand, vorzugsweise eine ophthalmische Linse, enthaltend das schwefelhaltige Polyharnstoffurethan nach einem der Ansprüche 1 bis 11.

**14.** Photochromer Gegenstand, enthaltend das schwefelhaltige Polyharnstoffurethan nach einem der Ansprüche 1 bis 11.

## Revendications

**1.** Poly(urée-uréthane) soufré, adapté pour présenter, à l'état au moins partiellement durci, un indice de réfraction, déterminé selon la norme ASTM D 542-00, d'au moins 1,57, un nombre d'Abbe, déterminé selon la norme ASTM D 542-00, d'au moins 32, et une masse volumique inférieure à 1,3 g/$cm^3$, qu'on a préparé en faisant réagir ensemble, dans un procédé mis en oeuvre « en un seul récipient » :

a) un polyisocyanate choisi parmi les diisocyanates aliphatiques, les diisocyanates cycloaliphatiques, les diisocyanates aromatiques, leurs dimères cycliques et leurs trimères cycliques, ainsi que les mélanges de tels composés,
b) une matière soufrée à hydrogène actif,
c) et un agent durcisseur aminé.

**2.** Poly(urée-uréthane) soufré, adapté pour présenter, à l'état au moins partiellement durci, un indice de réfraction, déterminé selon la norme ASTM D 542-00, d'au moins 1,57, un nombre d'Abbe, déterminé selon la norme ASTM D 542-00, d'au moins 32, et une masse volumique inférieure à 1,3 g/$cm^3$, qu'on a préparé en faisant réagir ensemble, dans un procédé mis en oeuvre « en un seul récipient » :

a) un polyisothiocyanate,
b) une matière à hydrogène actif, choisie parmi les composés à groupe(s) hydroxyle OH, les composés à groupe(s) sulfhydryle SH, et les composés comportant à la fois des groupes fonctionnels hydroxyle et sulfhydryle,
c) et un agent durcisseur aminé.

**3.** Poly(urée-uréthane) soufré conforme à la revendication 1, pour lequel ledit agent durcisseur aminé est un agent durcisseur aminé soufré.

**4.** Poly(urée-uréthane) soufré conforme à la revendication 1, pour lequel ledit agent durcisseur aminé est un mélange d'un agent durcisseur aminé et d'au moins un composé choisi parmi un polythiol et un polyol.

**5.** Poly(urée-uréthane) soufré conforme à la revendication 1, pour lequel la matière soufrée à hydrogène actif est un composé à groupe(s) sulfhydryle, de préférence un polythiol.

**6.** Poly(urée-uréthane) soufré conforme à la revendication 1, pour lequel la matière soufrée à hydrogène actif est un mélange d'un polythiol et d'un polyol non soufré.

**7.** Poly(urée-uréthane) soufré conforme à la revendication 5, pour lequel ledit polythiol est choisi parmi les polythiols aliphatiques, les polythiols cycloaliphatiques, les polythiols aromatiques, les polythiols polymères, les polythiols comportant des raccords de type éther, et les polythiols comportant un ou plusieurs raccord(s) de type sulfure.

**8.** Poly(urée-uréthane) soufré conforme à la revendication 5, pour lequel le polythiol comprend au moins l'un des composés suivants :

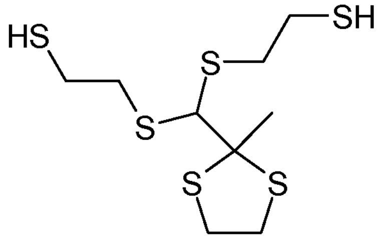

(IV'a)

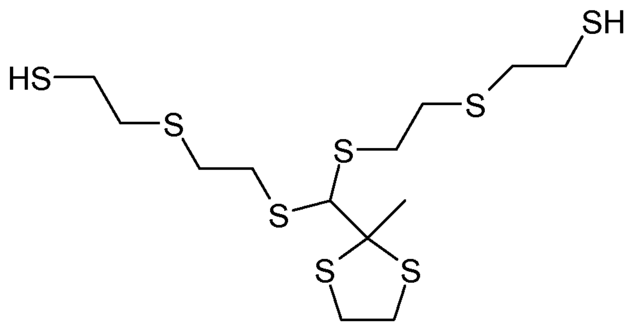

(IV'b)

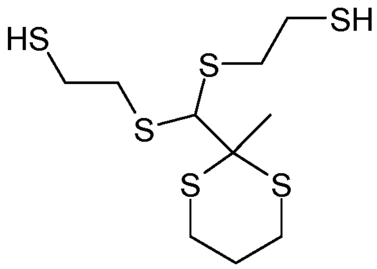

(IV'c)

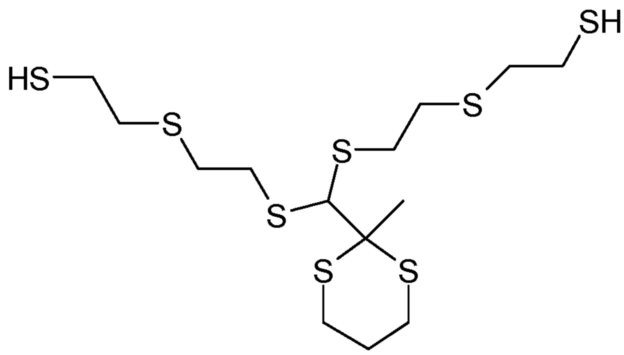

(IV'd)

**9.** Poly(urée-uréthane) soufré conforme à la revendication 5, pour lequel le polythiol comprend au moins un composé représenté par la formule structurale suivante :

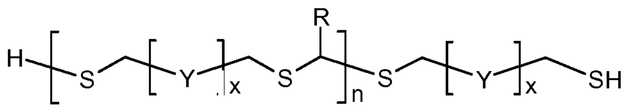

dans laquelle

- R peut représenter un groupe méthyle, acétyle, alkyle en $C_1$-$C_{10}$, cycloalkyle, aryl-alkyle, ou alkyl-carbonyle,
- Y peut représenter un groupe alkyle en $C_1$-$C_{10}$, cycloalkyle ou aryle en $C_6$-$C_{14}$, ou de formule $(CH_2)_p(S)_m(CH_2)_q$, $(CH_2)_p(Se)_m(CH_2)_q$ ou $(CH_2)_p(Te)_m(CH_2)_q$, où l'indice m peut être un nombre entier valant de 1 à 5 et les indices p et q peuvent chacun être un nombre entier valant de 1 à 10,
- l'indice n peut être un nombre entier valant de 1 à 20,
- et l'indice x peut être un nombre entier valant de 0 à 10.

**10.** Poly(urée-uréthane) soufré conforme à la revendication 5, pour lequel le polythiol comprend au moins l'un des

composés suivants : 1.

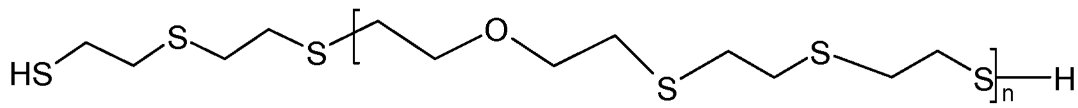

(IV'f)

n

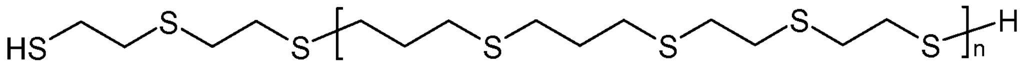

(IV'g)

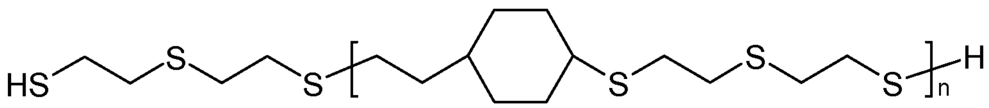

(IV'h)

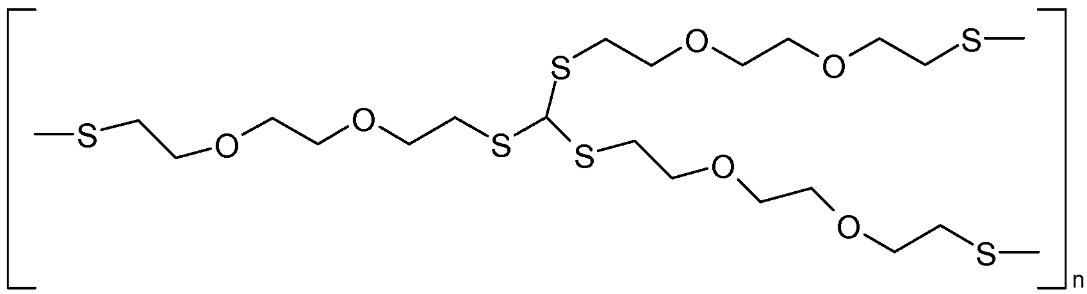

(IV'i)

chez lesquels l'indice n peut être un nombre entier valant de 1 à 20.

**11.** Poly(urée-uréthane) soufré conforme à la revendication 1, pour lequel la matière soufrée à hydrogène actif est un polysulfure à groupe(s) fonctionnel(s) hydroxyle, et de préférence, un polysulfure à groupe(s) fonctionnel(s) hydroxyle et comportant en outre un ou des groupe(s) fonctionnel(s) sulfhydryle.

**12.** Procédé de préparation d'un poly(urée-uréthane) conforme à la revendication 1 ou 2, qui comporte le fait de faire réagir, dans un procédé mis en oeuvre « en un seul récipient », le polycyanate (a), la matière à hydrogène actif (b) et l'agent durcisseur aminé (c).

**13.** Article optique, de préférence lentille ophtalmique, comprenant un poly(urée-uréthane) soufré conforme à l'une des revendications 1 à 11.

**14.** Article photochrome, comprenant un poly(urée-uréthane) soufré conforme à l'une des revendications 1 à 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2644007 A
- US 2680127 A
- US 6187444 B1
- US 4160853 A
- US 6509418 B1
- WO 03042270 A
- WO 0166623 A1
- US 5225472 A
- US 5693738 A
- US 3562172 A
- US 3578602 A
- US 4215010 A
- US 4342668 A
- US 5405958 A
- US 4637698 A
- US 4931219 A
- US 4816584 A
- US 4880667 A
- US 4818096 A
- US 3567605 A
- US 4826977 A
- US 5066818 A
- US 5466398 A
- US 5384077 A
- US 5238931 A
- US 5274132 A
- US 5429774 A
- US 3361706 A
- US 4931220 A
- US 4166043 A
- US 4367170 A


### Non-patent literature cited in the description


- **D.M. YOUNG ; F. HOSTETTLER et al.** Polyesters from Lactone. *Union Carbide F-40,* 147
- *CHEMICAL ABSTRACTS,* 96-27-5
- Ullmann's Encyclopedia of Industrial Chemistry. 1992, vol. A21, 673-674
- *CHEMICAL ABSTRACTS,* 101-02-0